# EUROPEAN PATENT APPLICATION

(11) **EP 4 765 143 A1**
(43) Date of publication of application: **24.06.2026**
(21) Application number: 24221228.0
(22) Date of filing: 18.12.2024
(51) Int. Cl.: G16H 20/60, G16H 20/70, G16H 50/30, G16H 40/67, A61B 5/00, H04W 4/02

(54) **IMPROVEMENTS IN DEMENTIA PREVENTION AND MITIGATION**

(71) Applicant: Berkeley Psychiatrists Ltd, London W1J 5BF (GB)
(72) Inventor: de Waal,, Hugo Eduard, London, W1J 5BF (GB); Devvaal,, Alp, London, W1J 5BF (GB)
(74) Representative: Carpmaels & Ransford LLP

(57) **Abstract**

Systems and methods are provided for assisting patients in preventing or delaying the onset of dementia, for aiding in the treatment of dementia and its symptoms, and for facilitating improved care of patients with dementia. One example comprises receiving, for a patient, a severity score for each of a plurality of remediable dementia risk factors; computing, for the patient, an impact-weighted score for each remediable dementia risk factor based on the received severity scores and a plurality of respective predetermined impact weights; computing, for the patient, a corrigibility-weighted score for each remediable dementia risk factor based on the impact-weighted scores and a plurality of respective corrigibility weights; and generating a mitigation plan for the patient, wherein generating the mitigation plan comprises ordering the plurality of remediable dementia risk factors by their respective corrigibility-weighted scores.

## Description

### Field of Invention

The present invention relates to systems and non-therapeutic methods for assisting patients in preventing or delaying the onset of dementia, for aiding in the treatment of dementia and its symptoms, and for facilitating improved care of patients with dementia.

### Background

Dementia is a syndrome associated with a plurality of neurodegenerative diseases, the most common subtypes of dementia being Alzheimer's disease, vascular dementia, Lewy body dementia, frontotemporal dementia, and mixed dementia. It is currently the seventh leading cause of death worldwide, according to the World Health Organisation. About one in eleven people over the age of 65 are thought to have dementia. At time of writing, dementia affects about one million people even just in the UK alone, and this figure continues to increase as the population's life expectancy increases.

Persons suffering from dementia can display a range of symptoms. They may become agitated and restless. They may experience disrupted thought patterns and a decline in their cognitive abilities, struggling to solve problems, understand their environment, pay attention, or take on new information. They may experience decreased motivation and lose interest in activities they previously enjoyed, and may not want to get out of bed. They can become increasingly forgetful, and struggle to recall memories from their life; they may get lost in their own neighbourhood, or forget to take medications, carry out household chores, or perform tasks associated with personal care or hygiene. They may fail to remember names or recognise faces of family members and friends, and become verbally or physically aggressive towards them. They may experience visual hallucinations, or hear voices. They may experience urinary or faecal incontinence. Patients with late-stage dementia may have trouble sleeping, and may struggle even with tasks such as eating or walking.

The symptoms of dementia usually get worse over time. There is no known cure.

There are a variety of known risk factors associated with dementia. Some of these risk factors are irreversible, such as aging, or having experienced a head injury. Other ones of these risk factors, however, can be reversed to at least some extent. For example, a patient who is obese may decrease their dementia risk by losing weight through a combination of diet and exercise. Even risk factors such as visual or auditory impairment can be remedied to at least some extent via a range of interventions e.g., by prescribing glasses or hearing aids, by corrective surgery, and so forth.

Some attempts have been made by clinicians to help reduce dementia risk among the population, e.g., by making generic recommendations to eat healthily, exercise often, socialise regularly, and avoid smoking or drinking excessively. However, it would be desirable if more could be done to reduce the severity of patients' dementia, postpone its onset and worsening, and lower the likelihood of developing it in the first place.

It is therefore the aim of the present invention to provide improvements in how dementia is prevented, mitigated and managed.

### Summary

In a first aspect of the invention, there is provided a computer program comprising instructions which, when the program is executed by a computer, cause the computer to carry out steps comprising: receiving, for a patient, a severity score for each of a plurality of remediable dementia risk factors; computing, for the patient, an impact-weighted score for each remediable dementia risk factor based on the received severity scores and a plurality of respective predetermined impact weights; computing, for the patient, a corrigibility-weighted score for each remediable dementia risk factor based on the impact-weighted scores and a plurality of respective corrigibility weights; and generating a mitigation plan for the patient, wherein generating the mitigation plan comprises ordering the plurality of remediable dementia risk factors by their respective corrigibility-weighted scores.

In a further aspect of the invention, there is provided a device comprising a processor and a memory, the memory comprising instructions which, when executed by the processor, cause the processor to carry out steps comprising: receiving, for a patient, a severity score for each of a plurality of remediable dementia risk factors; computing, for the patient, an impact-weighted score for each remediable dementia risk factor based on the received severity scores and a plurality of predetermined impact weights; computing, for the patient, a corrigibility-weighted score for each remediable dementia risk factor based on the impact-weighted scores and a plurality of corrigibility weights; and generating a mitigation plan for the patient, wherein generating the mitigation plan comprises ordering the plurality of remediable dementia risk factors by their respective corrigibility-weighted scores.

In a further aspect of the invention, there is provided a computer-implemented method comprising the following steps: receiving, for a patient, a severity score for each of a plurality of remediable dementia risk factors; computing, for the patient, an impact-weighted score for each remediable dementia risk factor based on the received severity scores and a plurality of predetermined impact weights; computing, for the patient, a corrigibility-weighted score for each remediable dementia risk factor based on the impact-weighted scores and a plurality of corrigibility weights; and generating a mitigation plan for the patient, wherein generating the mitigation plan comprises ordering the plurality of remediable dementia risk factors by their respective corrigibility-weighted scores.

Advantageously, by taking into account not only the severity scores but the impact weights as well, the mitigation plan can be generated to prioritize addressing those risk factors which are the most impactful on the patient's dementia risk. This prevents a possible situation where a patient directs most or all of their efforts into addressing a risk factor for which they personally exhibit a high severity score but which ultimately still does not carry as high a dementia risk to them as some other risk factor (for which they exhibit a lower severity score but which may have a greater impact).

Moreover, by factoring in the corrigibility weights of different risk factors for the patient when generating their mitigation plan, the resultant plan can prioritize addressing those risk factors which present the easiest "early gains" or "low hanging fruit", and can thereby improve the rate at which their dementia risk is lowered per unit effort expended at each stage of the mitigation plan. This prevents a possible situation where a patient directs most or all of their efforts into addressing a risk factor which has a large impact on their likelihood of developing dementia, but which they may be unable personally to remedy effectively (or at least, which will consume so much time, energy and effort for the patient to address that these resources could have been spent more efficiently on addressing a different risk factor with a higher corrigibility weight for that patient).

Optionally, the severity scores represent, for each remediable dementia risk factor, an extent to which the patient exhibits the risk factor.

Optionally, generating the mitigation plan comprises one or more of: generating, for one or more of the remediable dementia risk factors, a recommendation of a lifestyle change for the patient to mitigate the respective one or more risk factors based on the ordering and/or the respective corrigibility-weighted scores; generating an action plan based on the ordering and based on one or more identified actions for addressing at least one risk factor of the plurality of remediable dementia risk factors; or determining one or more prioritised risk factors among the remediable dementia risk factors based on the ordering and/or the respective corrigibility-weighted scores.

Advantageously, this enables the mitigation plan to be tailored to the patient's unique situation, accounting for the effect of each risk factor both i) objectively and ii) in relation to the other risk factors.

Optionally, receiving the severity scores comprises: receiving, for the patient, a severity score for each of a plurality of dementia risk factors, said plurality including each of the remediable dementia risk factors and one or more irremediable dementia risk factors; and filtering the plurality of received severity scores to retain only the severity scores for the remediable dementia risk factors.

Advantageously, constraining the severity/corrigibility analysis to only those risk factors which are remediable reduces unnecessary consumption of computational resources such as memory, processing power, and screen space, whilst providing a more practical and more accurate estimation of dementia risk, risk factor effects, and suitable mitigations.

Optionally, the remediable dementia risk factors include one or more of: hearing loss, LDL cholesterol level, social isolation, depression, anxiety, physical inactivity, diabetes, smoking, hypertension, vision loss, obesity, and alcohol use.

Optionally, the predetermined impact weights are provided in a ratio of: about 7 for hearing loss; about 7 for LDL cholesterol level; about 5 for social isolation; about 3 for depression; about 3 for anxiety; about 2 for physical inactivity; about 2 for diabetes; about 2 for smoking; about 2 for hypertension; about 2 for vision loss; about 1 for obesity; and about 1 for alcohol use.

Advantageously, the present inventors have found that the above ratio provides the impact-weighted scores that most accurately model the risk factors' individual contributions to dementia risk.

Optionally, the impact weights represent, for each remediable dementia risk factor, the statistical contribution of said risk factor to a likelihood of the patient developing dementia.

Optionally, the impact weights are derived from statistical population data.

Advantageously, deriving the impact weights from statistical population data results in the calculated impact-weighted scores providing an accurate reflection of which risk factors can be seen empirically among the population to lead to greater dementia risk.

Optionally, the corrigibility weights represent the extent to which the respective factors are capable of correction for the patient.

Optionally, the corrigibility weights comprise, for each remediable dementia risk factor, a factor for a level of psychological effort and/or a factor for a level of logistical feasibility associated with adoption of an intervention by the patient to mitigate said risk factor.

Advantageously, factoring in the level of psychological effort associated with adoption of an intervention to mitigate a (or each) risk factor enables a plan to be generated that prioritises a low-effort intervention having a given impact over a high-effort intervention having the same or similar impact. This helps to avoid a situation where a patient's finite reserves of willpower and energy are inefficiently invested into a high-effort intervention when a low-effort (for the patient in question) intervention would have had an equal or greater impact on reducing their dementia risk.

Advantageously, factoring in the level of logistical feasibility associated with adoption of an intervention to mitigate a (or each) risk factor enables a plan to be generated that prioritises a feasible intervention having a given impact over an infeasible intervention having the same or similar impact. This helps to avoid a situation where a patient is presented with a mitigation plan that is based on interventions the patient will be unable to implement (or unable to implement except with great logistical difficulty).

Optionally, the factor for the level of psychological effort and/or the factor for the level of logistical feasibility are input by the patient or a care provider thereof, or are predetermined.

Optionally, the steps further comprise outputting to a display one or more of: the generated mitigation plan; the received severity scores for one or more of the remediable dementia risk factors; the calculated impact-weighted scores for one or more of the remediable dementia risk factors; the calculated corrigibility-weighted scores for one or more of the remediable dementia risk factors; a total remediable risk score based on adding the severity scores for each of the plurality of remediable dementia risk factors; a total impact-weighted risk score based on adding the impact-weighted scores for each of the plurality of remediable dementia risk factors; the received severity scores for one or more of the irremediable dementia risk factors; and a total risk score based on adding the severity scores for each of the remediable dementia risk factors and each of the irremediable dementia risk factors.

Advantageously, displaying this information enables a patient, carer, or clinician to understand and contextualise the patient's likelihood of developing dementia (or worsening dementia), the extent to which this likelihood can be remedied or reversed, and what steps can be taken to remedy or reverse it - including, where relevant, the additional context provided by the impact weights and/or corrigibility weights.

Optionally, said outputting comprises generating and rendering one or both of: a chart representing the severity scores for each of the remediable dementia risk factors; and a graph representing a change over time for the severity score of each of one or more of the remediable dementia risk factors.

Advantageously, this form of output enables a patient, carer, or clinician to understand the severity score for each risk factor in context relative to the other risk factors and/or relative to the same risk factor at different points in time.

Optionally, the steps further comprise: receiving, for the patient, an updated severity score for one or more selected risk factors of the plurality of remediable dementia risk factors; computing an impact-weighted score for each selected risk factor based on the one or more updated severity scores and the plurality of impact weights; and computing a total impact-weighted risk score based on adding the impact-weighted scores for each of the selected risk factors to the impact-weighted scores for each of the remaining remediable dementia risk factors.

Advantageously, computing this total impact-weighted risk score enables a patient, carer, or clinician to keep track of the patient's current total impact-weighted dementia risk even as their individual severity scores continue to change (e.g., as a response to successful implementation of a lifestyle change or other intervention intended to combat dementia risk).

Optionally, the steps further comprise providing an output to a display based on: said computed total impact-weighted risk score; and a second total impact-weighted risk score based on adding the impact-weighted scores for each of the plurality of remediable dementia risk factors.

Advantageously, this enables the new or ongoing total impact-weighted risk score to be understood in the context of the original or previous total impact-weighted risk score.

Optionally, the steps further comprise: generating a hypothetical reduced severity score for one or more selected risk factors of the plurality of remediable dementia risk factors; computing an impact-weighted score for each selected risk factor based on the one or more hypothetical reduced severity scores and the plurality of impact weights; and computing a hypothetical total impact-weighted risk score based on adding the impact-weighted scores for each of the selected risk factors to the impact-weighted scores for each of the remaining remediable dementia risk factors.

Advantageously, computing this hypothetical total impact-weighted risk score enables a patient, carer, or clinician to explore the potential effect that could be achieved in terms of dementia risk reduction if they were to implement a lifestyle change or other intervention successfully enough to reduce one or more severity scores to a certain value.

Optionally, the steps further comprise providing an output to a display based on: said computed hypothetical total impact-weighted risk score; and a second total impact-weighted risk score based on adding the impact-weighted scores for each of the plurality of remediable dementia risk factors.

Advantageously, this enables the hypothetical total impact-weighted risk score to be understood in the context of the patient's actual or current total impact-weighted risk score.

Optionally, the received severity scores comprise: severity scores received from electronic medical records; and/or severity scores received based on input to a graphical user interface by the patient or a caregiver thereof.

Advantageously, receiving the severity scores from electronic medical records can improve consistency and reduce human error, whilst using a GUI input can lead to scores which are more up-to-date and accurate, and which may be received more quickly.

Optionally, one or more of the severity scores are generated based on: biometric data sensed by a wearable device; and/or location data and/or GPS data determined by a wearable device or mobile device.

Advantageously, this can eliminate human error completely regarding scores for certain risk factors, and can yield an objective, empirical, reliable and trustworthy measure of the degree of exposure a patient is likely to have to each risk factor.

Optionally, receiving the severity scores comprises generating a severity score for a social isolation risk factor, wherein generating said severity score comprises: receiving patient-routine data comprising location data and/or GPS data determined by a wearable device or mobile device of the patient; deriving one or more patient-routine metrics from the received patient-routine data; and optionally generating and outputting one or more patient prompts based on the derived patient-routine metrics and receiving one or more patient responses to said patient prompts.

Advantageously, this permits a dementia risk to be tracked and mitigated based on analysis of a degree of a patient's social isolation via their location/GPS data. Generating and outputting the prompts and receiving the responses allows fine-tuning of the social isolation severity score using responses to customised prompts that are tailored to be specific to a patient's observed routine and patterns therein.

Optionally, generating the mitigation plan further comprises: for each candidate activity of a plurality of candidate activities, comparing location data for said candidate activity against the derived patient-routine metrics to determine a fit score; and incorporating one or more of the candidate activities into the generated mitigation plan based on their determined fit score and the generated severity score for the social isolation risk factor.

Advantageously, this provides an improved mitigation plan, since the patient can be provided with suggestions of activities for reducing social isolation which are specifically relevant to their own unique routine and patterns of behaviour, and which are appropriate for their current level of social isolation.

Optionally, the plurality of candidate activities are generated based on the patient-routine data and/or the derived patient-routine metrics.

Advantageously, using the patient-routine data/metrics enables candidate activities to be proposed and considered that are likely to be relevant to the patient's routine.

Optionally, generating the mitigation plan comprises generating an intervention for at least one risk factor, based on one or both of: the severity score for said risk factor; and an amount of time in which the severity score for said risk factor has remained unchanged.

Advantageously, this enables the generated plan to be commensurate with the severities of the different risk factors associated with a patient, and to include interventions which are proportionate to patient's previous success or failure in mitigating each risk factor.

Optionally, the steps further comprise: detecting attempts by the patient to access or view a patient data item; computing a frequency or volume of the detected attempts; comparing said frequency or volume to a predetermined threshold; and upon determination that the frequency or volume exceeds the predetermined threshold, triggering an anti-obsession action.

Advantageously, this has been found by the present inventors to reduce the risk that a patient will begin to exhibit obsessive behaviour with respect to their data items, which is particularly important for the psychological health of persons suffering from dementia.

Optionally, the patient data item comprises one or more of: one of the received severity scores; one of the computed impact-weighted scores; one of the computed corrigibility-weighted scores; the total remediable risk score; the total impact-weighted risk score; the total risk score; the hypothetical total impact-weighted risk score; the chart; or the graph.

Optionally, the anti-obsession action comprises one or more of: transmitting an alert to a clinician, outputting an alert to the patient, blocking the attempt, or locking out the patient.

Advantageously, alerting, blocking or locking out the patient can help them to recognise patterns of obsessive behaviour when using the present invention and to break from these patterns; alerting the clinician can improve their understanding of the patient's state and symptoms and accordingly to provide improved clinical care.

Optionally, the severity scores are received by a server device, the impact-weighted scores are computed by the server device, the corrigibility-weighted scores are computed by the server device, and the mitigation plan is generated by the server device; and the steps further comprise transmitting the mitigation plan from the server device to a client device.

Advantageously, such a division of processing steps yields a more efficient use of computing resources and allows data from a wider range of sources to be collated and deployed when determining the patient's severity scores.

Optionally, the steps comprise repeating said receiving the severity scores, computing the impact-weighted scores, computing the corrigibility-weighted scores, and generating the mitigation plan for each patient of a plurality of patients; and the steps further comprise: receiving outcome data for each patient of the plurality of patients; generating a training dataset by matching mitigation plans or interventions thereof to the outcome data; and training a supervised machine learning model to map mitigation plans or interventions thereof to predicted outcomes using the training dataset.

Advantageously, such training results in the supervised machine learning model being capable of understanding at a population level which interventions provide the most benefit for which patients, such that less successful mitigation plan recommendations can be replaced with more successful ones.

Optionally, the steps further comprise using the trained supervised machine learning model to predict an outcome of each of one or more proposed mitigation plans or interventions thereof, and optionally to output a recommended mitigation plan or intervention thereof based on the predicted outcomes.

Advantageously, such use of the trained model results in selection of the plan or intervention that can be expected to result in the best patient outcome based on all available training data.

In a further aspect of the invention, there is provided a computer program comprising instructions which, when the program is executed by a computer, cause the computer to carry out steps comprising: receiving first patient-routine data during a validation period, the first patient-routine data comprising location data and/or GPS data determined by a wearable device or mobile device of the patient; computing an expected pattern or range for the patient for each of a plurality of patient-routine metrics, based on the first patient-routine data; receiving second patient-routine data after expiry of the validation period, the second patient-routine data comprising location data and/or GPS data determined by a wearable device or mobile device of the patient; detecting a deviation from the expected pattern or range in one or more of the patient-routine metrics based on the second patient-routine data; and transmitting an alert to the patient and/or a clinician based on the detected deviation.

In a further aspect of the invention, there is provided a device comprising a processor and a memory, the memory comprising instructions which, when executed by the processor, cause the processor to: receive first patient-routine data during a validation period, the first patient-routine data comprising location data and/or GPS data determined by a wearable device or mobile device of the patient; compute an expected pattern or range for the patient for each of a plurality of patient-routine metrics, based on the first patient-routine data; receive second patient-routine data after expiry of the validation period, the second patient-routine data comprising location data and/or GPS data determined by a wearable device or mobile device of the patient; detect a deviation from the expected pattern or range in one or more of the patient-routine metrics based on the second patient-routine data; and transmit an alert to a clinician based on the detected deviation.

In a further aspect of the invention, there is provided a computer-implemented method comprising: receiving first patient-routine data during a validation period, the first patient-routine data comprising location data and/or GPS data determined by a wearable device or mobile device of the patient; computing an expected pattern or range for the patient for each of a plurality of patient-routine metrics, based on the first patient-routine data; receiving second patient-routine data after expiry of the validation period, the second patient-routine data comprising location data and/or GPS data determined by a wearable device or mobile device of the patient; detecting a deviation from the expected pattern or range in one or more of the patient-routine metrics based on the second patient-routine data; and transmitting an alert to a clinician based on the detected deviation.

Advantageously, determining abnormalities and changes in a patient's routine or social behaviour in this way permits dementia risk to be detected early, so that measures can be timely put in place to mitigate it.

Optionally, the patient-routine metrics comprise one or more of: a route taken between two locations; an amount of time taken to travel between two locations; a frequency with which a location is visited; an amount of time spent at a location; a day or plurality of days on which a location is visited; and a time or range of times at/in which a location is visited.

In a further aspect of the invention, there is provided a non-transitory computer-readable storage medium containing instructions which, when read by a computer, cause the computer to perform the method of the above aspects.

Other objects, advantages and novel features of the present invention will become apparent from the following detailed description of one or more preferred embodiments when considered in conjunction with the accompanying drawings.

### Brief Description of the Drawings

The present invention has been described below purely by way of example with reference to the accompanying drawings in which:
Fig. 1 depicts a system for dementia prevention/mitigation in accordance with embodiments of the present invention;
Fig. 2 is a component diagram of a typical device for use in the system of Fig. 1;
Fig. 3 graphically illustrates a process for generating a mitigation plan for a patient based on a plurality of severity scores in accordance with various embodiments of the present invention;
Figs. 4a, 4b, 4c and 4d depict exemplary GUI elements for display to a user such as a patient, carer or clinician in accordance with embodiments of the present invention;
Fig. 5a depicts an exemplary GUI element for a patient to input data for deriving a severity score for a dementia risk factor; Fig. 5b depicts an example of an anti-obsession feature in accordance with at least one embodiment of the present invention;
Fig. 6 depicts patient-routine data in the form of location data for a patient in accordance with embodiments of the present invention;
Figs. 7a and 7b depict more exemplary GUI elements for display to a user such as a patient, carer or clinician in accordance with embodiments of the present invention;
Fig. 8 depicts an exemplary supervised machine learning model suitable for mapping mitigation plans or interventions thereof to predicted outcomes in accordance with at least one embodiment of the present invention; and
Figs. 9a and 9b are flowcharts depicting first and second methods of the present invention.

### Detailed Description

The detailed description set forth below provides information and embodiments of the disclosed technology with sufficient detail to enable those skilled in the art to practice the disclosure. It should be recognised that except where explicit recognition is provided to the contrary (for instance by their inclusion in an independent claim of the appended claims), none of these components or features should be taken as essential for implementing the present invention.

### Introduction

The present invention provides, inter alia, systems and methods for providing patients with a bespoke, tailor-made dementia risk mitigation plan. The plan avoids the risk of a patient wasting energy, losing interest and commitment, or wasting resources on less relevant risks. It can achieve this by classifying each of a plurality of reversible risk factors in terms of its severity (e.g., using a suitable scale or scoring system); weighting the identified reversible risks' impact in order of relevance (e.g., based on population attributable factors or "PAF"s); and assigning a final weighting based on the patient's environment, habits and circumstances.

Risk factors present in an individual may be identified from their history, from investigations, and/or via other suitable means described herein. Any suitable number of remediable risk factors may be considered - in various examples herein a set of twelve remediable risk factors is used, though it should be understood that this reflects merely one illustrative and non-limiting implementation of the invention. Using a severity scale for each risk factor, severity scores can be assigned for each identified risk factor. In various (non-limiting) examples herein, this scale may be an integer scale from 0 to 5 (inclusive). These severity scores can optionally be output (e.g., displayed) to a user (e.g., the patient, their carer, their clinician, etc). For instance, they can be listed in order of high-to-low severity.

For each risk factor, an appropriate impact weight can be identified. The impact weight can be based on a PAF as described herein. Each severity score can be weighted using its associated impact weight, for example by multiplying the two together, to get impact-weighted severity scores. Each impact weight could be, for instance, some percentage amount. The impact-weighted severity scores can optionally be output (e.g., displayed) to a user (e.g., the patient, their carer, their clinician, etc). For instance, they can be listed in order of high-to-low impact-weighted severity.

A user (e.g., a patient, or a carer or clinician who may optionally be aided by the patient) can assign a corrigibility score for each risk factor. In various (non-limiting) examples herein, this scale may be an integer scale from 2 to 6 (inclusive) - in particular, as described in more detail elsewhere herein, two factors may be provided and added together, each factor being an independent score from 1 to 3 inclusive. In other examples, simply one factor or the other could be used on its own as the corrigibility weight, i.e., providing an integer scale from 1 to 3. The corrigibility-and-impact-weighted severity scores (which for the sake of conciseness will be indicated as simply "corrigibility-weighted" severity scores) can optionally be output (e.g., displayed) to a user (e.g., the patient, their carer, their clinician, etc). For instance, they can be listed in order of high-to-low corrigibility-weighted severity - that is, with risk factors that are easier for the patient to remedy placed above risk factors having the same or similar impact-weighted severities but which are harder for the patient to remedy.

The set of corrigibility-weighted scores, and/or a list of remediable dementia risk factors sorted/ordered on the basis of these scores, can be used as the basis for a mitigation plan. A mitigation plan may be a custom plan for an individual to remedy or otherwise manage the onset of dementia and/or their risk thereof. Having a list of dementia risk factors weighted in order of severity, impact, and ease of mitigation is highly advantageous for compiling a mitigation plan, and moreover this list can be shared (e.g., displayed) with the patient or their caregiver/clinician to help improve provision of person-centred care, both reactively and proactively (i.e., prophylactic care to avoid or delay the onset of dementia). In this way, an ease-of-mitigation hierarchy is established to assist the patient in focusing on the easiest, but most impactful, risk factors.

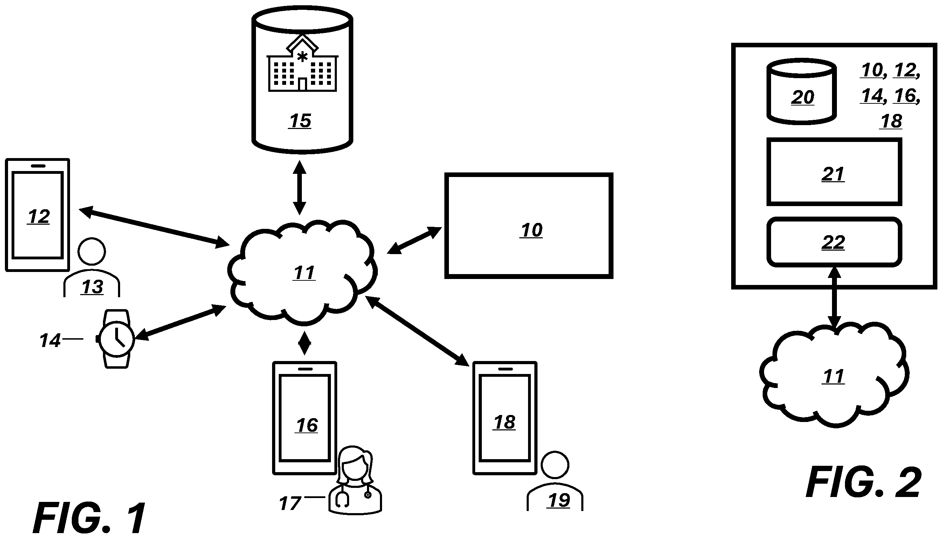

Referring to Fig. 1, there is provided an exemplary system for dementia prevention and mitigation. The system may in general comprise a processing device 10 and one or more devices associated with a patient 13 (in the illustrated example, a mobile device 12 and a wearable device 14). The system may optionally further comprise one or more of: a data store 15, one or more devices associated with a clinician 17 (mobile device 16 in the illustrated example), and one or more devices associated with a carer 19 who may be e.g., a professional carer or a friend or family member of patient 13 (mobile device 18 in the illustrated example).

Processing device 10 may be a computing device such as a server. Processing device 10 may be communicatively coupled with one or more of mobile devices 12, 16, 18 (and/or wearable device 14) in accordance with a server-client architecture as will be familiar to those skilled in the art. A mobile device may include e.g., a smartphone (such as a member of the Apple iPhone series, Samsung Galaxy series, Google Pixel series, or the like) a tablet computer (such as a member of the Apple iPad series, Samsung Galaxy Tab series, or the like).

Wearable device 14 may include e.g., a smartwatch or other activity tracking watch (Apple Watch, Samsung Galaxy Watch, Google Pixel Watch, Fitbit Versa or Fitbit Sense), a fitness tracker, a "smart glasses" device, smart headphones or earbuds, smart rings (Oura Ring, Motiv Ring, Ringly) or any other wearable device (e.g., a wearable glucometer device). Data store 15 may comprise a relational database managed by a relational database management system (e.g., MySQL, PostgreSQL, Microsoft SQL Server), a non-relational (e.g., NoSQL) database managed by a non-relational database management system (e.g., MongoDB, DynamoDB), a data warehouse (Amazon Redshift, Google BigQuery, Snowflake), or other suitable data store. Data store 15 may comprise electronic medical record (EMR) data, including EMR data for patient 13.

The exemplary system of Fig. 1 is by no means the only implementation by which the benefit of the present invention may be realised. As will be evident to those skilled in the art, any one or more of the mobile devices 12, 16 and 18 in the example depicted in Fig. 1 may be replaced by some other device(s) such as a desktop computer, laptop computer, tablet computer or other suitable alternative. Alternatively, any one or more of patient 13, clinician 17 and carer 19 may use a mobile device in addition to a second device of any of the types mentioned above. Whilst there may be specific advantages to using a plurality of devices as depicted in Fig. 1, the present invention may ultimately be put into effect using just a single device, which need not even necessarily be a mobile device.

Each of processing device 10, mobile device 12, data store 15, mobile device 16 and mobile device 18 may be in communication with each other via a network 11 (e.g., the internet) and are illustrated as such in Fig. 1, although it will be recognised by those skilled in the art that other networked configurations of these devices are possible. As just one example, in some embodiments patient device 12 and clinician device 16 may both be connected in some way to processing device 10, but patient device 12 and clinician device 16 need not share a direct connection or be connected by the internet to each other.

Referring to Fig. 2, additional components of one of the individual devices (such as processing device 10, wearable device 14 or one of mobile devices 12, 16, 18) are depicted, including a processor 21, memory 20 and communication interface 22. The processor 21 is configured to obtain computer executable code from the memory 20 and execute the computer executable code to perform the processes described herein. The other device(s) may each also be configured with the same type of components in the same fashion as depicted in Fig. 2. In some embodiments, any one or more of the processing device 10, wearable device 14 or mobile devices 12, 16, 18 may additionally be configured with components for user interaction such as a display and/or a user input device configured to receive user input into processor 21 (e.g., a touch screen, keyboard/keypad, mouse/trackpad, or the like). However, it will be recognised that such user-facing features may not always be essential to realise the benefits associated with the present invention.

It will be appreciated that the transmission of data among components of the system of Fig. 1 may occur in a variety of specific ways, many of which are essentially functionally equivalent for the purposes of the present invention. For example, data may be transferred from one device or component to another device or component over network 1 via "push"-style proactive sending steps by the transferring component, or via "pull"-style steps carried out on the processor of the receiving component, such as repeated polling of the transferring component to determine whether new data is available and ready to be transferred. Networking may be implemented using a layered model such as the TCP/IP model in accordance with any suitable set of selected application, transport, internet and data link layer protocols as will be known to those skilled in the art.

In at least one embodiment, steps of the present invention may be shared, divided or distributed between one or more components in the system, e.g., between processing device 10 and patient device 12. For instance, processing device 10 may be configured to receive severity scores for at least some of the remediable dementia risk factors from patient device 12 (and/or one or more of wearable device 14, data store 15, clinician device 16 or carer device 18) which have been transmitted to processing device 10 over network 11. Processing device 10 may then compute the impact-weighted scores and corrigibility-weighted scores for said risk factors and generate a mitigation plan for the patient in accordance with the present invention, then transmit the generated mitigation plan over network 11 to patient device 12 (and/or one or both of devices 16, 18).

In an alternative embodiment, all of the above steps up to but not including generating the mitigation plan may be carried out by processing device 10, but then processing device 10 may transmit e.g., the computed set of corrigibility-weighted scores (or an ordered list of risk factors based thereon) over network 11 to patient device 12 (16/18). Indeed, any other suitable "division of labour" may be used, with steps shared between patient device 12 and processing device 10 as appropriate.

One particularly advantageous implementation of the present invention involves the use of a mobile application or "app". In this implementation, patient device 12 is a mobile device such as a smartphone, onto which software is downloaded and installed. This software is configured either to perform all of the steps of the present invention on its own, or to connect to an external device (e.g., processing device 10, embodied as a server) across network 11 (e.g., the internet) in order to facilitate the steps of the present invention to be performed by the two devices in combination.

The software ("app") may connect to the server via any suitable interface, such as an application programming interface (API). Processing device 10 may be configured to run software of its own facilitating the performance of the steps of the present invention in combination with patient device 12. The app may be downloaded via a suitable marketplace service such as the Apple App Store or Google Play Store. Advantageously, this enables the benefits of the present invention to be realised by any user in possession of a smartphone, without them necessarily needing to visit a clinician or medical facility in person.

### Overview of Scoring, Weighting, & Generating a Mitigation Plan

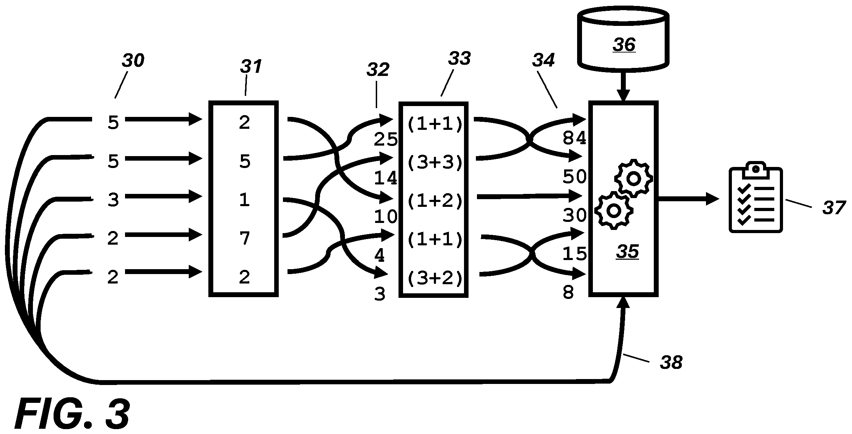

A worked example of the present invention will now be explained, with reference to Fig. 3. It should be noted that although the illustrated example is limited to using a set of just five remediable dementia risk factors for the sake of clarity and conciseness, in practice, any suitable number (e.g., twelve) may be used. Moreover, the illustrated impact weights and corrigibility weights, as well as the method by which weighting is applied, are not limiting.

As shown in Fig. 3, the severity scores 30 for a patient in a set of five remediable dementia risk factors may be as follows (on a scale of zero to five):

| | |
|---|---|
| Physical inactivity | 5 |
| Social isolation | 5 |
| Obesity | 3 |
| LDL cholesterol | 2 |
| Hypertension | 2 |

Immediately, it is possible to compute a total remediable/reversible risk score (for dementia) for the patient by summing all of these severity scores 30. One can also compute a total risk score by adding severity scores 30 to a set of one or more severity scores for other (non-remediable) dementia risk factors. In the present case, the total remediable risk score obtained by adding severity scores 30 is 5+5+3+2+2 = 17 out of a maximum of 25. The total (remediable/non-remediable/overall) score may be output to a user, e.g., the patient. The output can involve e.g., a graph or chart.

After severity scores 30 are received (e.g., simply identified, or loaded into memory), a step is performed in which impact weights 31 for the risk factors are used to compute impact-weighted scores 32. The number of impact weights 31 may be the same as the number of severity scores 30, i.e., the number of risk factors. In this example, weighting severity scores 30 using impact weights 31 comprises multiplying them together. In the illustrated example, impact weights 31 are as follows (on a scale of 1 to 7):

| | |
|---|---|
| Physical inactivity | 2 |
| Social isolation | 5 |
| Obesity | 1 |
| LDL cholesterol | 7 |
| Hypertension | 2 |

Thus, the impact-weighted scores 32 for the patient are as follows:

| | |
|---|---|
| Physical inactivity | 5 x 2 = 10 |
| Social isolation | 5 x 5 = 25 |
| Obesity | 3 x 1 = 3 |
| LDL cholesterol | 2 x 7 = 14 |
| Hypertension | 2 x 2 = 4 |

Immediately, it is again possible to compute a total impact-weighted remediable, non-remediable, and or total risk score; the total (remediable/non-remediable/overall) score may be output to a user, e.g., the patient, and the output can involve e.g., a graph or chart.

After impact-weighted scores 32 are computed, they are used in combination with corrigibility weights 33 to compute corrigibility-weighted scores 34. The number of corrigibility weights 33 may be the same as the number of severity scores 30, i.e., the number of risk factors. In the illustrated example, each corrigibility weight 33 comprises two factors - a first factor for a level of psychological effort associated with adoption of an intervention by the patient to mitigate the respective risk factor, and a second factor for a level of logistical feasibility associated with adoption of an intervention by the patient to mitigate the respective risk factor. These psychological and logistical factors are explained in more detail elsewhere herein. In the illustrated example, corrigibility weights 33 are as follows (on a scale of 1+1 to 3+3):

| | | | |
|---|---|---|---|
| Physical inactivity | Psychological: 1 | Logistical: | 2 |
| Social isolation | Psychological: 1 | Logistical: | 1 |
| Obesity | Psychological: 3 | Logistical: | 2 |
| LDL cholesterol | Psychological: 3 | Logistical: | 3 |
| Hypertension | Psychological: 1 | Logistical: | 1 |

Thus, the corrigibility-weighted scores 34 for the patient are as follows:

| | |
|---|---|
| Physical inactivity | 10 x (1+2) = 30 |
| Social isolation | 25 x (1+1) = 50 |
| Obesity | 3 x (3+2) = 15 |
| LDL cholesterol | 14 x (3+3) = 84 |
| Hypertension | 4 x (1+1) = 8 |

Although the five remediable risk factors may have initially had a first order of severity score 30 before either set of weights 31, 33 were applied (from "physical inactivity" and "social isolation" as most severe, to "LDL cholesterol" and "Hypertension" as least severe), a new order has now been derived which places LDL cholesterol first, followed by social isolation, physical inactivity and obesity, and then finally hypertension last. The LDL cholesterol risk factor has been moved up the list by virtue of being easy for the patient to mitigate and relatively impactful on dementia risk compared to the other risk factors.

A plan generating component 35 (e.g., a software component) then uses corrigibility-weighted scores 34 to generate a mitigation plan 37 for the patient. Generating mitigation plan 37 may involve ordering the remediable dementia risk factors by their corrigibility-weighted scores 34. For instance, generating mitigation plan 37 can involve recommending a lifestyle change(s) for the patient to mitigate one or more risk factors based on their position in the ordering, and/or their corrigibility-weighted scores 34. Lifestyle changes associated with risk factors for which the patient has a higher corrigibility-weighted score 34 may precede those associated with risk factors for which the patient has a lower corrigibility-weighted score 34. Additionally or alternatively, generating the plan can involve generating an action plan based on the ordering and on one or more identified actions for addressing the relevant remediable dementia risk factors.

Each lifestyle change or action may simply refer to a goal or objective for the patient to complete e.g., "reduce smoking to 20 cigarettes per day", or it may include elements specifying a recommended means of completion (e.g., "use nicotine-free vape to reduce smoking to 20 cigarettes per day") and/or a deadline or timeframe (e.g., "reduce smoking to 20 cigarettes per day before October 16th this year, to 10 per day before October 16th next year, to 0 per day before October 16th the following year, and maintain reduced consumption going forward").

Additionally or alternatively, one or more risk factors may be prioritized in the generated plan 37. This can be done based on the corrigibility-weighted scores 34 directly, or based on the order of the risk factors when ranked by these scores (i.e., based indirectly on the scores). Prioritizing a risk factor may involve increasing its importance (and/or the importance of measures for addressing it) in the mitigation plan 37. In general, a mitigation plan might require that steps be taken to achieve a goal or objective in relation to a higher-priority (i.e., higher-ranked) risk factor first before any suggestion or encouragement is provided to the patient to turn their attention to any lower-priority/lower-ranked risk factor (e.g., the next highest on the list). As an example, the plan may be generated to require that the patient lower their weight to below one hundred kilograms before moving ahead to a subsequent step of cutting down on alcohol consumption. Alternatively, the plan may permit or encourage the patient to take two or more actions/enact two or more lifestyle changes concurrently (in parallel), e.g., "Primary goal: reduce weight < 100kg; Secondary goal: cut alcohol to < 14 units/week".

The mitigation plan generator 35 may generate mitigation plan 37 with components (e.g., actions, goals, objectives, lifestyle changes) for each of the remediable dementia risk factors under consideration - in the example of Fig. 3, this would be a five-part plan for addressing the patient's inactivity, isolation, obesity, LDL cholesterol and hypertension. It is preferable that this is the case, i.e., the plan addresses all, or at least many, of the remediable dementia risk factors. However, the plan need not be so limited and may instead include components only for certain ones of the risk factors. As just one example, a threshold value for the corrigibility-weighted scores may be used to determine whether to include a mitigation plan component for each given risk factor at all - in the case of Fig. 3, taking a threshold of T=20 would result in a three-part mitigation plan recommending actions, changes or choices for addressing the patient's LDL cholesterol, social isolation and physical inactivity, but not necessarily their obesity or hypertension. In at least one embodiment, the plan 37 contains components for all of those risk factors whose severity score 30 was not zero, and only those risk factors.

As represented by arrow 38 in Fig. 3, plan generator 35 may optionally also take into account the values of the original severity scores 30 for one or more of the risk factors when generating mitigation plan 37. Where a mitigation plan 37 has components relating to each of a plurality of remediable risk factors, each individual component may be derived at least in part from its respective severity score 30. Optionally, the higher the severity score 30, the greater the magnitude or intensity the suggested countermeasure (change/action etc) for that risk factor may be. For instance, the part of mitigation plan 37 relating to a patient's obesity may vary depending on their severity score 30 for obesity: for a score of 1 or 2, the action may comprise simply minor dietary alterations and improvements; for 3 or 4, an Ozempic recommendation may be generated and output as the action; for 5, the action may comprise a surgical intervention (e.g., gastric band).

As shown in Fig. 3, the plan generator 35 may optionally also take into account any relevant stored historical data 36 for the patient when generating mitigation plan 37. This could comprise e.g., previous lifestyle changes that have been adopted by and/or recommended to the patient; previous severity scores entered by/for the patient; amounts of time for which one or more severity scores have remained unchanged; outputs of a machine learning algorithm such as the one described elsewhere herein; and so forth.

Mitigation plan 37 need not be output solely to the patient themself (or even to the patient at all). One or more (or all) of the parts of the plan may be output to the patient's clinician and/or carer in addition to, or instead of, the patient. This can provide better support for the patient in enacting lifestyle changes - moreover, the clinician can be notified of recommended pharmaceutical interventions directly, so that they can immediately begin consideration of whether they may be clinically appropriate, and start to prescribe them if so.

As an illustrative example, a mitigation plan 37 may be developed for the patient having the scores illustrated in Fig. 3. Plan generator 35 adds components for each of the five remediable risk factors to plan 37. The first (highest-priority) part of the plan is for addressing the patient's LDL cholesterol level; their severity score 30 for this risk factor is only 2, so the generated recommendation is limited to dietary and exercise-based recommendations. The second part of the plan is for addressing the patient's social isolation; their severity score 30 for this risk factor is 5, and has not yet responded to milder interventions (e.g., generic suggestions to improve socialisation), so the generated recommendation is a more direct and specific suggestion output to the patient and their carer comprising a recommended social activity they may enjoy based on patterns of routine observed from their GP/location data (as explained in more detailed elsewhere herein). The third part of the plan is for addressing the patient's physical inactivity; their severity score 30 for this risk factor is 5, although historical data shows that this score has fluctuated a lot in the past, with physical activity increasing significantly for the patient during summer months and reducing during winter. Based on this, in combination with the high severity score 30 for social isolation, a recommendation to exercise with a friend, partner or group is generated for this risk factor. The fourth part of the plan is for addressing the patient's obesity; their severity score 30 for this risk factor is only 1, so again the generated recommendation is limited to minor dietary improvements. The fifth (lowest-priority) part of the plan is for addressing the patient's hypertension; their severity score 30 for this risk factor is 2, recently reduced from a 3, and thus the generated recommendation in this part of the plan is limited to diet and exercise changes (with the recommendation that the patient take hypertensives being withdrawn from the patient and their clinician).

As another illustrative example, if a patient has a nonzero score for the "Physical Inactivity" risk factor, a proposed intervention may be generated according to a plurality of factors including: the patient's age and gender (e.g., collected by the patient's mobile device or at an intake interview); the patient's abilities, disabilities and personality traits (e.g., loaded from stored records of the patient's general history, generated/updated by a clinical psychologist); and the patient's previous physical activities and sports interests (e.g., loaded from stored records of a functional assessment generated/updated by an occupational therapist). The present invention can combine these factors with the severity score for the patient in this risk factor and formulate one or more suitable interventions or mitigation plans, taking into account the data gathered by all of the multidisciplinary team and taking into account the patient's personal characteristics and preferences. The invention can comprise either generating the entirety of the plan itself, or providing one or more suggestions, frameworks, outlines or baselines for a plan to be completed by one or more members of the team (e.g., a clinician).

A risk mitigation component for each risk factor can be informed by, and dependent on, the nature of the risk itself. Additionally, or alternatively, the component can be informed by, and dependent on, the severity score of the risk factor. The nature of the recommended intervention can additionally or alternatively depend on its impact-weighted and/or corrigibility-weighted score. It can depend on historical scores for that risk factor for the patient. It can depend on what interventions have been tried so far, how long they have been tried for, and the order in which they have been tried. Thus, interventions can be formulated by a software application and/or clinical team, taking into account the corrigibility-weighted scores (or at least the order they impose on the remediable risk factors), target scores the patient may wish to work towards, and a suitable timeline for completion of the intervention.

Further details of several aspects of the invention are set out below.

### Risk Factors & Severity Scores

Any suitable selection of remediable (and, where relevant, non-remediable) dementia risk factors may be used when implementing the present invention. What follows is a suggestion of one optional set of risk factors (and associated severity score scale for each risk factor) which has been found to work particularly well. Each risk factor in the below example has a score allocation from zero to five in ascending order of severity, based on widely used scales pertaining to that score which have undergone external validation (and as such, are not merely arbitrary scales). Alternative scales and score systems can be used.

Hearing loss is one example of a remediable dementia risk factor. Interventions for mitigating hearing loss include audiological interventions e.g., providing hearing aids to the patient. Data sources for a hearing loss severity score may include e.g., a result input by a clinician such as a GP or audiologist. Hearing loss can be scored as follows:
0: no hearing loss
1: mild hearing loss, difficulty hearing in the 26-40dB range
2: moderate hearing loss, difficulty hearing in the 41-55dB range
3: moderate-severe hearing loss, difficulty hearing in the 56-70dB range
4: severe hearing loss, difficulty hearing in the 71-90dB range
5: profound hearing loss, difficulty hearing in the 91+dB range

High LDL cholesterol is another example of a remediable dementia risk factor. Interventions for mitigating high LDL cholesterol include referral to a GP, dietary changes, and increasing exercise. Data sources for a high LDL cholesterol severity score may include e.g., an input by a clinician from a blood test. High LDL cholesterol can be scored as follows:
0: < 3.0 mmol/L, untreated
1: < 3.0 mmol/L, on statin
2: <3.0 mmol/L, on combination regimen*
3: >3.0 mmol/L, untreated
4: >3.0mmol/L, on statin
5: >3.0mmol/L, on combination regimen*
*a combination regimen may comprise a statin in combination with any of the following: ezetimibe, evolocumab, alirocumab, bempedoic acid, inclisiran

Social isolation is another example of a remediable dementia risk factor. Interventions for mitigating social isolation include the interventions generated and/or output by the anti-isolation feature described elsewhere herein, e.g., output by an app embodying the present invention. Data sources for a social isolation severity score may include e.g., scores derived using the Social and Emotional Loneliness Scale for Adults-S (15-105). More information about this scale can be found in DiTommaso, E., Brannen, C., & Best, L. A. (2004). Measurement and Validity Characteristics of the Short Version of the Social and Emotional Loneliness Scale for Adults. Educational and Psychological Measurement, 64(1), 99-119. https://doi.org/10.1177/0013164403258450. Social isolation can be scored as follows:
0: 15-29 out of 105, socially active
1: 30-44 out of 105, less active than before
2: 45-59 out of 105, small network out of choice
3: 60-74 out of 105, sometimes lonely, few friends
4: 75-89 out of 105, often lonely, few friends
5: 90-105 out of 105, lonely, no network, no confidants

Depression is another example of a remediable dementia risk factor. Interventions for mitigating depression include referral for clinical treatment (either with or without prescribing antidepressants) and referral for psychological therapy. Data sources for a depression severity score may include e.g., scores derived using the PHQ-9 scale (0-27). More information about this scale can be found in Kroenke, K., Spitzer, R. L., & Williams, J. B. (2001). The PHQ-9: validity of a brief depression severity measure. Journal of general internal medicine, 16(9), 606-613., and at https://patient.info/doctor/patient-health-questionnaire-phq-9. Depression can be scored as follows:
0: not depressed, 0-4 out of 27, not on antidepressant medication
1: mild, 5-9 out of 27, not on antidepressant medication
2: moderate, 10-14 out of 27, not on antidepressant medication
3: moderately severe, 15-19 out of 27, not on antidepressant medication
4: severe, 20-27 out of 27, not on antidepressant medication
5: 10-27 out of 27 AND on antidepressant medication

Anxiety is another example of a remediable dementia risk factor. Interventions for mitigating anxiety include referral for clinical treatment (either with or without prescribing anxiolytics) and referral for psychological therapy. Data sources for a anxiety severity score may include e.g., scores derived using the GAD-7 scale (0-21). More information about this scale can be found in Spitzer, R. L., Kroenke, K., Williams, J. B., & Löwe, B. (2006). A brief measure for assessing generalized anxiety disorder: the GAD-7. Archives of internal medicine, 166(10), 1092-1097., and at https://patient.info/doctor/generalised-anxiety-disorder-assessment-gad-7. Anxiety can be scored as follows:
0: not anxious, 0-4 out of 21, not on anxiolytic medication
1: mild, 5-9 out of 21, not on anxiolytic medication
2: 0-9 out of 21 AND on anxiolytic medication
3: moderate, 10-14 out of 21, not on anxiolytic medication
4: severe, 15-21 out of 21, not on anxiolytic medication
5: 10-21 out of 21 AND on anxiolytic medication

Physical inactivity is another example of a remediable dementia risk factor. Interventions for mitigating physical inactivity include increasing the frequency, duration and intensity of exercise and other physical activity; they may also include at least some of the interventions generated and/or output by the anti-isolation feature described elsewhere herein, e.g., output by an app embodying the present invention. Data sources for a physical inactivity severity score may include e.g., scores derived using the GPPAQ scale. More information about this scale can be found in Pearson, D., & Grace, C. (2012). General practice physical activity questionnaire. Weight Management: Wiley, 231-2., and at https://assets.publishing.service.gov.uk/media/5a7ac84ee5274a319e77ab03/GPPAQ-_guidance.pdf. Physical inactivity can be scored as follows:
0: active
1: moderate, recent
2: moderate, lifetime trait
3: little, recent
4: little, lifetime trait
5: totally inactive

Diabetes is another example of a remediable dementia risk factor. Interventions for mitigating diabetes include referral to a GP and dietary changes. Data sources for a diabetes severity score may include e.g., an input by a clinician from a blood test. Diabetes can be scored as follows:
0: HbA1c<6.0%
1: HbA1c<6.0%, diet
2: HbA1 c<6.0%, diet + medication
3: 6.0%<HbA1c<6.4%
4: HbA1c>6.5%, untreated
5: HbA1c>6.5%, treatment resistant

Nicotine dependence (also referred to herein as "smoking", though not necessarily limited to such tobacco-burning modes of nicotine consumption) is another example of a remediable dementia risk factor. Interventions for mitigating nicotine dependence include referral to a GP or appropriate smoking cessation service. Data sources for a nicotine dependence severity score may include e.g., scores derived using the Fagerstrom Test for Nicotine Addition (0-10). More information about this scale can be found in Heatherton, T. F., Kozlowski, L. T., Frecker, R. C., & FAGERSTROM, K. O. (1991). The Fagerström test for nicotine dependence: a revision of the Fagerstrom Tolerance Questionnaire. British journal of addiction, 86(9), 1119-1127., and at https://www.thecalculator.co/health/Fagerstrom-Test-For-Nicotine-Addiction-1037.html. Nicotine dependence can be scored as follows:
0: none
1: 1-2 out of 10, low
2: 3-4 out of 10, low to moderate
3: 5-6 out of 10, moderate
4: 7-8 out of 10, high
5: 9-10 out of 10, very high

High blood pressure (also known as hypertension) is another example of a remediable dementia risk factor. Interventions for mitigating high blood pressure include referral to a GP, dietary changes, and increasing exercise. Data sources for a high blood pressure severity score may include e.g., an input from a suitable wearable device such as a Samsung Galaxy Watch 7. High blood pressure can be scored as follows:
0: <120 systolic and <80 diastolic; not on hypertensives
1: 120-129 systolic and <80 diastolic; not on hypertensives
2: stage 1: 130-139 systolic or 80-89 diastolic; not on hypertensives
3: stage 2: >140 systolic or >90 diastolic; not on hypertensives
4: stage 3: >180 systolic or >120 diastolic; not on hypertensives
5: >= 130 systolic whilst on antihypertensives, or >= 80 diastolic whilst on antihypertensives

Vision loss is another example of a remediable dementia risk factor. Interventions for mitigating vision loss include interventions by an optician or ophthalmologist e.g., providing glasses to the patient, or ophthalmic surgery. Data sources for a vision loss severity score may include e.g., a result input by a clinician such as a GP, optician or ophthalmologist. Vision loss can be scored as follows:
0: normal
1: registered blind, from birth
2: mild visual impairment
3: moderate visual impairment
4: severe visual impairment
5: registered blind, acquired

Obesity is another example of a remediable dementia risk factor. Interventions for mitigating obesity include dietary changes, increasing exercise, or referral to a clinician for either medication (e.g., Semaglutide (sold under the brand name Ozempic)) or in some specific cases, surgery. Data sources for a high LDL cholesterol severity score may include e.g., inputs by the patient, a carer, and/or a clinician of either i) a BMI or ii) a height and/or weight for calculating. Obesity can be scored as follows:
0: 18.5<BMI<22.9
1: 23 < BMI 24.9
2: 25<BMI<29.9
3: 30<BMI<34.9
4: 35<BMI<39.9
5: BMI>40; severely obese

Alcohol use (including alcohol dependence) is another example of a remediable dementia risk factor. Interventions for mitigating alcohol use include e.g., outputting generic abstinence advice. Data sources for a high LDL cholesterol severity score may include e.g., scores derived using the AUDIT questionnaire (0-40). More information about this scale can be found in Bush, K., Kivlahan, D. R., McDonell, M. B., Fihn, S. D., Bradley, K. A., & Ambulatory Care Quality Improvement Project (ACQUIP. (1998). The AUDIT alcohol consumption questions (AUDIT-C): an effective brief screening test for problem drinking. Archives of internal medicine, 158(16), 1789-1795., and at https://auditscreen.org/check-your-drinking/. Alcohol use can be scored as follows:
0: none
1: 0-7 out of 40, low risk
2: 8-15 out of 40, moderate risk
3: 16-19 out of 40, higher risk
4: 20-29 out of 40, alcohol dependent
5: 30-40 out of 40, severe alcohol dependency

A total remediable severity score from 0 to 60 can be obtained by adding the severity scores for all of these remediable risk factors. The total remediable severity score can be assigned a category based on predefined thresholds or ranges, e.g., "Low Risk" for 0-20, "Moderate Risk" for 21-40, and "High Risk" for 41-60. This category and/or the total remediable severity score may be output via a display (e.g., the screen of a mobile device) to a user (e.g., the patient, their carer, or their clinician).

In addition to the above remediable risk factors, embodiments of the present invention may process one or more non-remediable (i.e., irreversible) risk factors, such as the four described below. Because these risk factors are irreversible, it is contemplated that their severity scores be removed or discounted (e.g., by performing a step of filtering out these scores) from the overall mitigation plan generation process depicted in Fig. 3. Indeed, these non-remediable risk factors may not even be assigned impact weights or corrigibility weights at all, since there may be no benefit to doing so. Nevertheless, these non-remediable risk factors can be included to inform the patient about their specific risk of developing dementia (and/or their carer, and/or their clinician). The severity scores for the non-remediable risk factors still contribute to the patient's overall dementia risk.

Amyloid score on a PET or CT scan is one example of a non-remediable dementia risk factor. Amyloid score can be scored as follows (note that scores of 1, 2 and 3 are deliberately not assigned):
0: BAPL0: no amyloid
4: BAPL1: minor amyloid load
5: BAPL2: significant amyloid load

The APOE gene (which encodes the apolipoprotein E protein) is another example of a non-remediable dementia risk factor. The APOE gene can be detected with genetic testing and can be scored as follows (note that scores of 1, 2 and 3 are deliberately not assigned):
0: ε2/ε2; ε2/ε3; ε3/ε3
4: ε3/ε4; 3x risk
5: ε4/ε4; 16x risk

Family history is another example of a non-remediable dementia risk factor. Family history can be scored as follows:
0: no family history
1: one family member, not 1st degree
2: one family member, 1st degree
3: >1 family member; not 1st degree
4: >1 family member; 1st degree
5: strong family history/autosomal dominant ("AD")

Head injury is another example of a non-remediable dementia risk factor. Head injury can be scored as follows:
0: no history
1: minor head injury, one-off
2: major head injury, one off, e.g. road traffic accident
3: major, prolonged recovery; or more than one head injury
4: frequent head injuries over <20 yrs (e.g. sports)
5: frequent head injuries over >20yrs (e.g. sports)

A total severity score from 0 to 80 can be obtained by adding the severity scores for all of the remediable risk factors to all of these non-remediable risk factors. The total severity score can be assigned a category based on predefined thresholds or ranges, e.g., "Low Risk" for 0-20, "Mild Risk" for 21-40, "Moderate Risk" for 41-60, and "High Risk" for 61-80. This category and/or the total severity score may be output via a display (e.g., the screen of a mobile device) to a user (e.g., the patient, their carer, or their clinician).

### Impact Weights

Each impact weight is a weighting added to each remediable risk factor's severity score, based on that risk factor's individual contribution to developing dementia. These weights may be expressed e.g., as percentages. The impact weights may be based on meta-analyses of population studies, and may for instance comprise or be derived from a PAF for the risk factor (or another suitable metric). As just one example, taking the PAF as the impact weight for each remediable dementia risk factor, impact weights may be assigned as follows:

| | |
|---|---|
| Hearing loss | 7% |
| High LDL cholesterol | 7% |
| Social isolation | 5% |
| Depression | 3% |
| Anxiety | 3% |
| Physical inactivity | 2% |
| Diabetes | 2% |
| Smoking | 2% |
| Hypertension | 2% |
| Visual loss | 2% |
| Obesity | 1% |
| Excessive alcohol | 1% |

Since the population attributable factors are used as the impact weights, the above weights represent the statistical situation whereby the number of people developing dementia overall would decrease by 1% if obesity were eliminated in full across the entire population; the number of people developing dementia overall would decrease by 2% if diabetes were eliminated in full across the entire population; the number of people developing dementia overall would decrease by 3% if depression were eliminated in full across the entire population; and so forth for the other remediable risk factors. Thus, a higher impact weight represents a greater contribution to dementia risk, and a lower impact weight represents a lesser contribution to dementia risk.

The above impact weights are supported e.g., in Livingston, G., Huntley, J., Liu, K. Y., Costafreda, S. G., Selbaek, G., Alladi, S., ... & Mukadam, N. (2024). Dementia prevention, intervention, and care: 2024 report of the Lancet standing Commission. The Lancet, 404(10452), 572-628.

### Corrigibility Weights

Each corrigibility weight is a further weighting informed by the individual's circumstances and preferences in terms of recommended mitigation activities. Because of the corrigibility factors, the mitigation plan is generated in a way which is unique to a patient's specific personal situation. The patient can be guided (e.g., by their mobile device, such as by an app thereon) to identify, for each remediable risk factor, how easy it would be for them either to improve/enhance existing habits, or to adopt an activity as a new habit, as part of a remediation plan. The corrigibility weights therefore enable the patient to influence the hierarchy of priorities in the generated mitigation plan. Risks can be scored higher if the patient judges a particular mitigation countermeasure to be less onerous, such that the list shows risk factors with those which represent 'easy gains' or `low hanging fruit' featuring at the top of the list. The corrigibility-weighted scores can be used to derive what is effectively a 'to-do' list of items in descending priority, starting with those that are easiest to tackle.

The greater the corrigibility weight, the greater the extent to which the respective factor is capable of correction for the patient, and the lesser the corrigibility weight, the lesser the extent to which the respective factor is capable of correction for the patient. Applying the corrigibility weighting to the impact-weighted scores has been found by the present inventors to enhance the sense of achievement felt by the patient as they advance through the mitigation plan, and to motivate them to persist with it.

Though any suitable corrigibility weighting scheme may be used, one embodiment contemplated is for each risk factor to be assigned a two-part corrigibility weight associated with adoption of an intervention by the patient to mitigate said risk factor. The first part of this corrigibility weight is a factor for a level of psychological effort, and the second part is a factor for a level of logistical feasibility. Applying the two-part corrigibility weight to an impact-weighted score comprises adding the two factors together and then multiplying the impact-weighted score by the result. The two factors can both be scored using the following scale:
1: "Considerable effort and support needed"
2: "Some effort needed"
3: "Not a problem"

Psychological effort can be determined based on one or more of: a level of willpower required by the patient to implement an intervention; an amount of mental effort required to implement the intervention; an amount of self-control required to implement the intervention; an amount of discipline required to implement the intervention; and so forth.

It must be stressed that the prioritisation of low-psychological-effort interventions in a mitigation plan for reducing dementia risk has a specific technical effect going beyond the mere satisfaction of subjective user preferences. "Ego depletion", the process by which individuals' levels of self-control or willpower are drained by expending energy, even if only mental energy, is a well-studied psychological phenomenon supported by empirical evidence - the idea that self-control or willpower is a finite resource that can be lost and replenished, as is intuitively obvious to many people, is consistent with results of psychological research. Accordingly, given that all mental effort investment comes at a cost, it is objectively more desirable for the patient's finite resources to be spent on those interventions where this investment will go the furthest. By having the first items tackled in the mitigation plan be those which deplete the least psychological effort, the patient is able to work further through the mitigation plan than would otherwise be possible (e.g., if all of their effort had been invested on a single very high-effort intervention). More information about ego depletion can be found e.g., in Baumeister, R. F., Bratslavsky, E., Muraven, M., & Tice, D. M. (2018). Ego depletion: Is the active self a limited resource?. In Self-regulation and self-control (pp. 16-44). Routledge.

As an example, a patient may have no objection at all to attending appointments with a GP, an optician and an audiologist to address their hypertension, vision loss and hearing loss, and so may score a "3" for the psychological effort factor of the corrigibility weight for each of these risk factors. However, the same patient may score a "1" for the psychological effort factor associated with the alcohol use risk factor (e.g., because they are addicted to alcohol, or simply because they enjoy drinking in excess very much). In this case, even though the patient's alcohol use may be more pronounced (having a higher severity score than the other factors), the plan may be generated to prioritize the other factors ahead of it because this will be a more efficient use of their willpower and mental effort. A particular patient may be unable to make and stick to a plan involving cutting out alcohol, cigarettes, high-fat/high-sodium processed foods etc and exercising every day, but they are much more likely to be able to introduce one of these changes at a time.

Logistical feasibility can be determined based on one or more of: whether a proposed intervention is capable of being implemented at all by a patient; an amount of time required to implement the intervention; a number of barriers to implementation; the severities of such barriers; a logistical or monetary cost to implement the intervention; an availability of specialist staff or resources to the patient; and so forth.

As an example, a first patient who is paraplegic and technology-literate might give a lower score (e.g., 1 or 2) for interventions intended to address physical inactivity and obesity via increased exercise than a second patient who is able-bodied but does not own (and/or cannot use) a computing device; conversely, the same first patient may give a much higher score (e.g., 3) for interventions aimed at addressing depression or anxiety because they have access to online support and can speak to a psychotherapist remotely, in contrast to the second patient.

It is advantageous that a corrigibility weight be assigned for every risk factor, though not necessarily essential - if a given risk factor lacks a specific assigned corrigibility weight, a predetermined default corrigibility weight may be used instead.

### GUIs & Displays

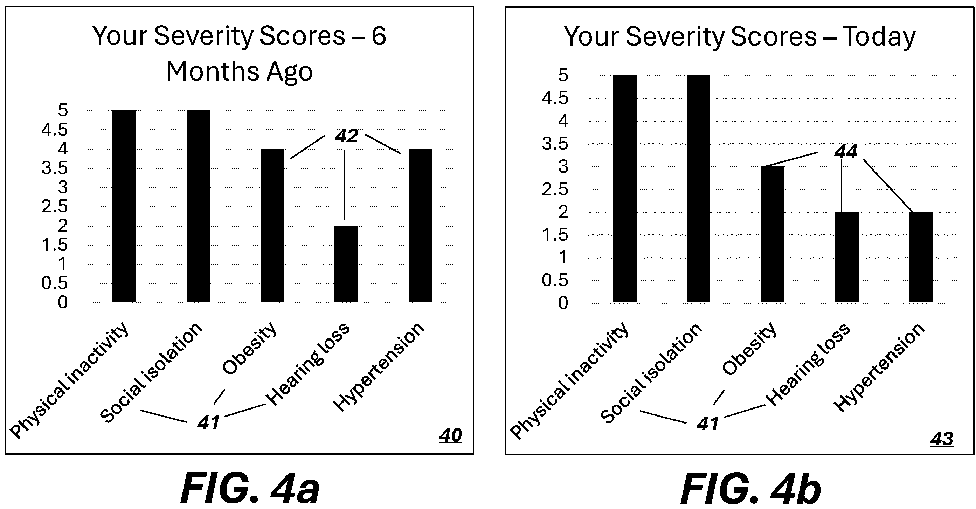

Referring now to Figs. 4a-4d, various exemplary graphical user interface (GUI) outputs are illustrated. Any one or more of these outputs, elements thereof, or combinations thereof may be displayed via suitable display means (e.g., a screen of a mobile device) to the patient and/or another user (such as their clinician or carer).

As shown in Figs. 4a and 4b, steps according to the present invention can comprise outputting severity scores for one or more (or all) of the risk factors (e.g., all of the remediable risk factors). This outputting may comprise generating and rendering e.g., a chart 43 representing the current (most recently updated) severity scores 44 for each risk factor 41 (Fig. 4b), and/or a chart 40 representing the historical severity scores 42 for each risk factor 41 at some previous point in time e.g., a specific date/time, or a specific amount of time before the present e.g., *n* months (Fig. 4a). The current and historical severity scores 44, 42 may be graphically displayed separately (e.g., in different panes or windows, or at different times) or together (e.g., using an overlay, transparency, dashed lines, or the like).

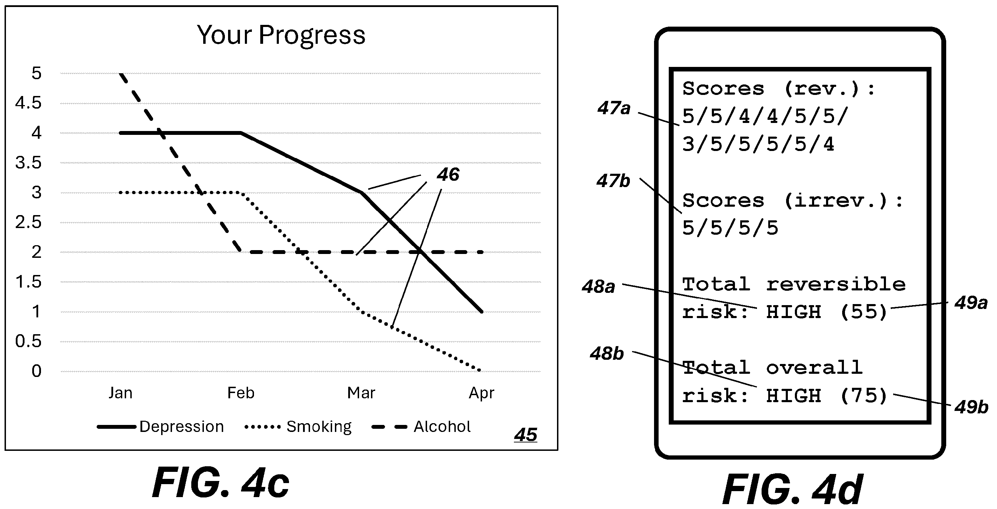

As shown in Fig. 4c, in addition to graphically illustrating their current severity scores, the present invention may comprise graphically illustrating changes in one or more severity scores (i.e., severity scores for one or more remediable dementia risk factors) over a period of time. The steps can comprise outputting received severity scores for a risk factor by generating and rendering a graph 45 representing a change over time for one or more severity score(s) 46. Multiple scores 46 can be rendered simultaneously for the same window or time period using any suitable technique e.g., coloured or dashed lines, transparency or overlays, and so forth.

This can advantageously optimise an individual's engagement and adherence with their risk mitigation plan by presenting graphical illustrations of the change in their severity scores over time (e.g., as the mitigation plan is followed). This enables a patient and/or clinician uniquely to track change directly as the patient deploys the recommended mitigation plan, thereby presenting the patient with a quantified representation of the success of their actions, enabling both an instantaneous and an ongoing, easily accessible biofeedback. This in turn fosters the patient's understanding of how risks are decreasing at any point in time in a quantified way. The scores at first assessment can be illustrated for the patient in graph 45 to capture and visualise over time, thereby reinforcing the element of biofeedback. This is highly advantageous for optimising continued patient engagement.

As shown in Fig. 4d, even without rendering any graphical elements such as charts or graphs, data pertaining to the various risk factors may still be output to any suitable user. For example, severity scores, impact-weighted scores, and/or corrigibility-weighted scores can be output as textual or numerical data in a graphical or text-based interface of a device. This can include severity scores for remediable or reversible risk factors (47a), severity scores for non-remediable or irreversible risk factors (47b), a category 48a or numerical value 49a for a total remediable risk score, or a category 48b or numerical value 49b for a total risk score.

The same steps used to determine the initial impact-weighted scores can also be used to determine updated impact-weighted scores as corresponding updated severity scores are received (e.g., from data streams such as sensors of a wearable device and/or GPS/location data from a mobile device or wearable device). Likewise, the same steps used to determine the initial corrigibility-weighted scores and/or mitigation plan can be used to determine updated corrigibility-weighted scores and/or mitigation plans. These updated scores can be rendered and displayed graphically e.g., as described above.

Similarly, the same steps used to determine the initial impact-weighted scores can also be used to compute a hypothetical effect on the patient's impact-weighted scores (and hence total impact-weighted score representing an overall dementia risk) that may be expected if the patient were to follow certain specific mitigation options, scenarios or interventions. That is, for each component of the generated mitigation plan, the steps of the invention may further comprise presenting the potential effect of various hypothetical scenarios or severity score changes on individual impact-weighted scores as well as overall impact-weighted scores.

Moreover, since certain interventions may affect the severity score for more than one of the remediable dementia risk factors (e.g., dietary changes may impact LDL cholesterol, diabetes, hypertension and obesity; joining a running club may impact LDL cholesterol, social isolation, physical inactivity and obesity; and so forth), a hypothetical effect of a single intervention (lifestyle change, action, objective etc) on each of a plurality of scores may be calculated and displayed to a patient/carer. These scores can again be the individual or total severity scores of the remediable risk factors, or the (individual or total) impact-weighted scores of the remediable risk factors.

These hypothetical impact-weighted scores can be displayed textually and/or graphically to a suitable user such as the patient or their carer. For example, a graphical display may indicate to the patient what would happen to their individual or total severity scores (and/or their individual or total impact-weighted scores) if they were to decrease their smoking from forty cigarettes a day to zero. This serves to enhance adherence and motivation further by clearly charting possible progress if certain mitigations are achieved.

### Smart Data Sources for Severity Scores

The severity scores for one or more of the dementia risk factors may be input by the patient's clinician and/or retrieved from stored EMR data. Candidate risk factors for this kind of input are e.g., LDL cholesterol, vision loss, amyloid score, and APOE. In accordance with the present invention, the severity scores are not restricted to being so input by the clinician or retrieved from storage, however, and indeed the present inventors have devised several ways in which alternative data sources may be used to improve the efficacy of the invention.

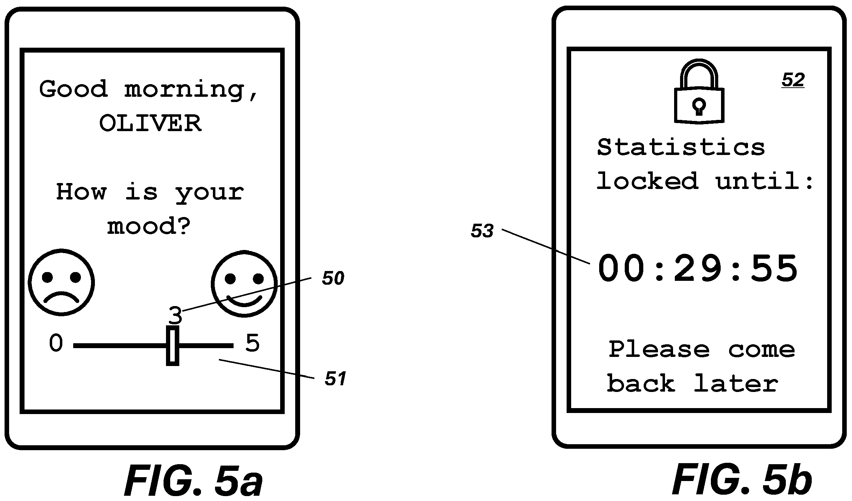

As one example, at least one severity score may be input by the patient themselves. As illustrated in Fig. 5a, a graphical user interface may prompt the patient to enter information relating to a specific risk factor into their mobile (or desktop) device, using suitable input means, in order to determine a corresponding severity score therefrom. Suitable input means may comprise one or more of a touchscreen, mouse, keyboard, trackpad, or the like. The patient may for instance enter the information by clicking and dragging to select a value 50 using a slider element 51 (as shown in Fig. 5a); alternatively, information may be input by entering alphanumeric characters, or by selecting an option from a dropdown list, or the like. Data may be input in the style of a kind of "online diary".

As another example, at least one severity score may be input by a carer of the patient. Again, suitable means may be used including a touchscreen, mouse, keyboard, trackpad, or the like, as part of a mobile or desktop computing device. This can advantageously improve the accuracy of answers and ease with which they are input, because the patient may (particularly if they are in an advanced stage of dementia) either struggle with the physical act of inputting a response to a prompt into their device, or may input a response to a prompt which their carer knows not to be accurate (e.g., due to the patient mis-remembering).

In particularly advantageous embodiments, sensor data (e.g., real-time sensor data from a wearable device) may be used to derive the severity score for at least one of the dementia risk factors. For example, blood pressure (for tracking hypertension) can be determined directly from certain smartwatch devices or wearable cuffs. Alcohol intake may be determined directly by wearable biosensors that can detect the presence of ethanol in sweat. Glucose levels (for a diabetes severity score, say) can be determined directly using a continuous glucose monitor (CGM) device. Physical (in)activity can be determined directly using fitness wearables or smartwatches, or even to some extent by the accelerometer found in many common models of smartphone, or other mobile devices. Moreover, data pertaining to some risk factors may be monitored indirectly (e.g., tracking smoking via changes in heart rate or blood oxygen levels; tracking alcohol consumption based on heart rate variability or sleep patterns/sleep quality; or tracking tissue composition (fat/muscle) using bioelectrical impedance analysis to inform a severity score for obesity.

Additionally or alternatively, location data and/or GPS data (e.g., real-time data) may be used to derive the severity score for at least one of the dementia risk factors. "GPS data" can include any geolocation data derived using a GPS receiver or GPS chip built into a mobile device or wearable device, based on transmissions received from a plurality of (at least four) satellites. By processing the received raw data, the patient's position (or more accurately, the position of the device they carry) can be determined to within 5-10 metres. The term also includes assisted GPS (A-GPS) geolocation data. "Location data" can include additional geolocation data which need not itself be GPS-based (or at least not wholly so), e.g., Wi-Fi positioning data derived using known hotspot locations, cellular network location data derived using triangulation between phone masts, Bluetooth data, crowdsourced location data, and so forth.

In at least one embodiment, the remediable dementia risk factors include a "social isolation" risk factor, and the severity score for said risk factor is generated based at least in part on location data and/or GPS data. In at least one embodiment, the remediable dementia risk factors include a "physical inactivity" risk factor, and the severity score for said risk factor is generated based at least in part on location data and/or GPS data. In at least one embodiment, both social isolation and physical inactivity severity scores are included and are generated based at least in part on location data and/or GPS data.

Using objectively measured (e.g., sensed/biometric/location/GPS) data in this way advantageously avoids even more of the aforementioned pitfalls associated with inputs received from the patient or their carer; since the patient may not always be relied upon to answer questions completely accurately, and since their carer (if they even have one) may not know where they are and what they are up to at every hour of the day (particularly if the patient is still in very early-stage dementia and able to operate with some independence), relying exclusively on these users can lead to severity scores (or corrigibility weights) being reported inaccurately, and hence can lead to suboptimal mitigation plan generation. By receiving and processing sensed/biometric/location/GPS data to derive at least some of the severity scores, an objective, factual and accurate measure of each associated risk factor can be acquired, and an improved mitigation plan consequently generated.

In some embodiments, initial values of severity scores may be provided based on user-entered data alone, without using sensor data (e.g., wearable sensor data), but with such data then being used to obtain subsequent severity score values later, i.e., after the initial assessment has concluded. In some embodiments, initial values of severity scores may be provided based on user-entered data alone, without using location/GPS data (or without using specific/precise/accurate location/GPS data, or without using both kinds of data), but with such data then being used to obtain subsequent severity score values later, i.e., after the initial assessment has concluded.

### Anti-Obsession Feature

Referring now to Fig. 5b, certain embodiments of the present invention are described which comprise an anti-obsession feature, designed to detect and/prevent obsessive patterns of the behaviour by the patient in relation to their individual or total scores (including e.g., severity scores and/or impact-weighted scores). It is known that patients with dementia can at times attempt to cope in general by overfocusing on specific matters. This behaviour can lead to emotional distress, physical harm, or neglected self-care, and can thus significantly impact their quality of life, safety, and overall well-being, as well as the well-being of caregivers.

To discourage such obsessive behaviours, the patient's access to certain data (e.g., scores), menus, graphs, charts and the like may be limited. Acceptable limits can be predefined/hard-coded or set solely by a clinician; for the sake of improved autonomy of the patient they may advantageously be agreed by the patient at the start of a clinical engagement, before they start using the features of the present invention. The system can then log attempts by the patient to get around these deliberate limitations, and any attempts beyond a certain predefined threshold (in terms e.g. of frequency or volume) can trigger a suitable action. In one example, the triggered action is to generate and transmit an alert to the clinician (since if the log shows an increase in attempts by the patient to view their data compared to previous usage, this may represent a diagnostic relevant deterioration).

Various embodiments of the triggered anti-obsession action are contemplated. In an example depicted in Fig. 5b, a frequency or volume of attempts by the patient to view their latest severity scores for the dementia risk factors can trigger a lock-out event, causing an interface 52 to be displayed to the patient for all further attempts until the end of a cool-down period 53. In some embodiments, an alert can be output to the patient or clinician, or the attempt may simply be blocked either with or without further explanation being given.

### Anti-Isolation Feature

Anti-isolation steps of the present invention may proceed by receiving first patient-routine data during a validation period, then receiving second patient-routine data after expiry of the validation period and detecting whether there has been a deviation from an expected pattern or range of one or more patient-routine metrics (which are computed based on the first patient-routine data). An initial patient and/or carer input, e.g., via a questionnaire or other suitable interface, may optionally be used prior to the validation period to establish a general pattern of the patient's routine movements and activities.

The validation period may enable determination of margins of error regarding various statistics, e.g., a route taken between two locations, an amount of time taken to travel between two locations, a frequency with which a location is visited, an amount of time spent at a location, a day or plurality of days on which a location is visited, or a time or range of times at/in which a location is visited.

The end of the validation period (in which a pattern is established from the GPS and/or location data for the patient) can be based on a predetermined timespan in some embodiments. Alternatively, the present invention may comprise automatically determining an end of the validation period based on a detection that newly-received GPS/location data is no longer having any significant impact on the computed pattern/range of first patient-routine data (e.g., has had no significant impact for a certain minimum timespan e.g., the last 7 days). Determining a "significant" impact (or lack thereof) may comprise e.g., using a threshold value - for example, if a patient visits a location each day for some amount of time *t,* the validation period may end automatically when it is determined that the mean t value, taking into account all of the values for n over the last two weeks (say), has not moved by more than ten minutes. Other suitable metrics (e.g., route, travel time, visit frequency, visitation days/times) calculated statistics (e.g., median, first and third quartiles), suitable sliding window timeframes (other than two weeks), and suitable thresholds (other than ten minutes) will be apparent to those of ordinary skill in the art.

After validation, normal GPS/location monitoring can start and can continue for whatever period the patient desires, informed by the clinician's advice; any deviation outside of the established margins of error can then e.g., generate an alert to the clinician (and/or to the patient or carer, depending on predetermined preferences), for further interpretation. In general, unwanted and/or unplanned changes in established routines are known to indicate onset or worsening of dementia (i.e. they are of diagnostic value), to negatively impact the patient's general and cognitive functioning, to increase anxiety and the risk of other co-morbidities (depression, paranoid ideation etc), and to increase carer burden. Charting routines and detecting changes therein therefore provides useful "scaffolding" for the patient and assists in maintaining those routines that are deemed important by them and their carers/relatives. This in turn preserves their level of functioning and has a beneficial effect on the course of the dementia.

Determining patterns and ranges in the patient-routine metric(s) (such as route taken, time spent, and so forth) can involve inventorising the patient's movements using GPS/location data collected at identified times, determining the nature or identity of the geographic whereabouts, and linking these with identified interests and routines.

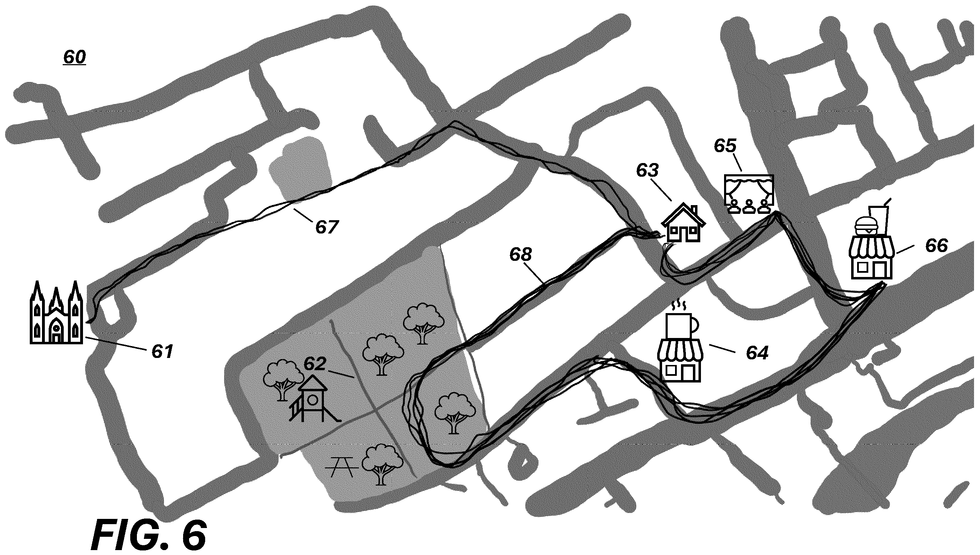

For example, with reference now made to Fig. 6, the patient-routine data 60 received for a patient may indicate a first pattern in the form of a commonly-repeated route 68 beginning and ending at the patient's place of residence 63. Further computed patterns and ranges may reflect e.g., that:
- The patient takes the route 68 almost every weekday;
- The patient typically leaves their place of residence 63 between 8:30 and 9:00 AM;
- The patient travels to a local park 62, taking seven to eight minutes to get there, and typically remains withing the bounds of park 62 for between thirty-five and forty-five minutes;
- The patient travels to a café 64, taking four to five minutes to get there, and typically stays there for between about one and one-and-a-half hours;
- The patient travels to a restaurant 66, taking six to seven minutes to get there, and typically stays there for between twenty and thirty minutes;
- The patient travels to an entertainment amenity 65, taking five to six minutes to get there, and typically stays there for not more than two hours; and
- The patient returns to place of residence 63 and takes three to four minutes to get there.

Instead of or in addition to amounts of time spent at each amenity or location, the anti-isolation feature may compute typical patterns or ranges in terms of the approximate times of day at which the patient arrives at/departs from said location, and/or in terms of times of day during which the patient is present within a specific area. For example, a further pattern may be determined in the form of route 67, which may be taken by the patient exclusively (though frequently) on Sundays, during which they leave place of residence 63 between 9:00 and 9:30 AM, travel to place of worship 61, where they remain for at least the window of 10:00 to 10:30 AM, and then spend at least the window of 12:00 to 1:00 PM having lunch back at place of residence 63.

As with detecting the end of the validation period, a range/pattern that is "typical" in the sense employed above can be determined based on standard statistical measures (e.g., between specific percentiles, within a given number of standard deviations, within a range that excludes outliers, or the like) and/or standard threshold-based techniques as will be known to those skilled in the art.

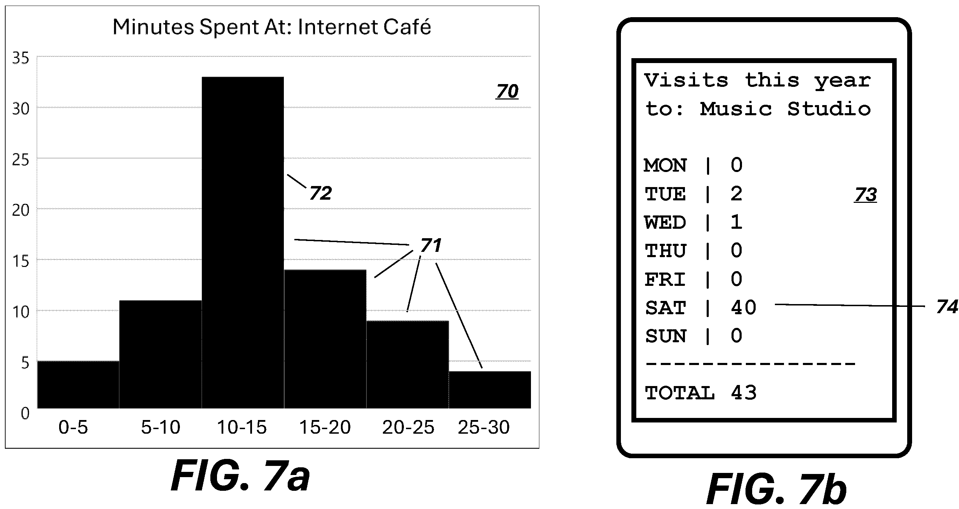

Referring to Figs. 7a and 7b, the patient-routine metrics can be determined by suitable binning or other statistical analysis and can optionally be used to provide an output to a patient, carer or clinician on a display accordingly. Fig. 7a illustrates values informing a patient-routine metric 70 (in this case, time spent at a location). Data representing individual daily measures of the amount of time spent can be binned into bins of a given width (in this case, four minutes), and the most populous bin 72 can be used to determine an expected range or pattern for that metric. As shown in Fig. 7b, in the case of a metric 73 comprising discrete and distinct values (e.g., a day of the week on which a venue is visited or an activity participated in), a modal value may be taken and used to establish the expected pattern/range in the first patient-routine data. In the illustrated example of Fig. 7b, this would involve "Saturday" being determined as the typical weekday on which the activity is performed. The raw patient-routine data, the patient-routine metrics derived therefrom, and/or the expected or typical patterns or ranges for these metrics, may be (but are not required to be) output to any one or more of the users mentioned above.

In some embodiments, the anti-isolation feature may generate and display prompts to request further information from the patient about the locations they have visited and their activities there ("Do you have lunch at McDonalds on Saturdays?"). These prompts may be determined at least in part based on the gathered patient-routine data and/or metrics thereof. Additionally or alternatively, the answers given by the patient to a first prompt may determine which prompts the patient is subsequently presented with - for instance, if the patient answers "Yes" to the prompt above, a subsequent prompt may be "Do you usually have lunch here alone?", and if the answer to this is "No", the next prompt may be "Does your company change, or do you frequently eat with the same people?" and so forth. In this way, a comprehensive and detailed description can be constructed of the salient features of the patient's social life and activities of daily living.

In various embodiments, the severity score(s) for at least one of the remediable dementia risk factors (e.g., social isolation) may be derived based at least in part on the data gathered with the anti-isolation feature - this includes the (first and/or second) patient-routine data, the patient-routine metrics derived from this data, and typical ranges/patterns thereof. It also includes information input by the patient in response to the prompts mentioned above. For instance, a patient who is observed to leave their home only very rarely may be assigned a higher severity score for social isolation than a patient whose location/GPS data and prompt responses indicate that they frequently participate in several social activities each day. Other remediable dementia risk factors such as physical inactivity may to some extent be influenced by this data gathered with the anti-isolation feature.

In various embodiments, the data gathered with the anti-isolation feature (including the (first and/or second) patient-routine data, the patient-routine metrics derived from this data, and typical ranges/patterns thereof) may be used for generating the mitigation plan, to at least some extent. Optionally, a threshold can be applied to the severity score for social isolation to determine whether the anti-isolation feature data should inform the mitigation plan (e.g., using this data to address the social isolation risk as part of the mitigation plan if its severity score is a three, four, or five out of five).

As a first example, anti-isolation feature data (such as GPS/location data and/or prompt responses) can inform the mitigation plan by generating recommendations based around cementing the patient's activities more firmly into a daily schedule ("You visited the Cabin Café three times this week - aim for four next week", "It looks like you have not been to Music Studio much this year - why not pay it a visit?").

As a second example, this anti-isolation feature data can inform the mitigation plan by generating recommendations based around expanding the patient's interests into similar interests in their vicinity (or online if possible and/or preferred), establishing a 'best fit' of proposed initiatives with the patient's already-established habitual activities. This may be done e.g., by generating or receiving some options for candidate activities, scoring them against derived patient-routine metrics (and/or responses to the abovementioned prompts) to determine a quality of "fit" to the patient for each option, and then using the best-fit candidate activity/activities to inform at least part of the generated mitigation plan. For instance, a patient who frequently spends time at art galleries can be given recommendations of classes and/or exhibitions to visit, or online courses to take part in, whereas a plan for a patient who visits lots of sports stadia may contain recommendations of football matches or related social activities.

### Improving Recommendations with Machine Learning

The present invention can include (or be configured to perform) one or more steps in which information is collected regarding how multiple patients' severity scores respond to the various interventions proposed in the mitigation plans generated according to the present invention. Such data-gathering can be used as training data for a machine learning model, which may eventually reveal as an outcome of training that certain interventions appear to result in better outcomes than others, so that epidemiological knowledge of what works best is generated, which then can be used as part of the present invention (e.g., in steps executed by the processing device) to change or replace less successful mitigation activities. This leads to an overall higher success rate as a result of the invention learning from the success/failure its own recommendations. In other words, by monitoring patient outcomes over time and correlating them against the interventions that were used by the respective patients prior to those outcomes being measured, the present invention can determine which plans and interventions thereof bring about the best outcomes.

Clinically speaking, although it may be possible to show a general beneficial effect of an intervention (e.g., decreasing obesity), the extent to which the particular risk factor is a constituent to the overall risk of the individual developing dementia or any other chronic disorder is unknowable, as there may be a plurality of known and unknown factors affecting each patient. The medical/scientific understanding of the total risk resulting from all (i.e., known and unknown) risk factors in one individual and their relative contribution to their future health is therefore limited. The machine learning function described herein will therefore generate new knowledge: not only can the size of the beneficial effect be charted for one individual through the changes in their scores, but in large numbers of users, such phenomena can be pooled, leading to new understanding at a 'population' level of what intervention generates the largest beneficial change.

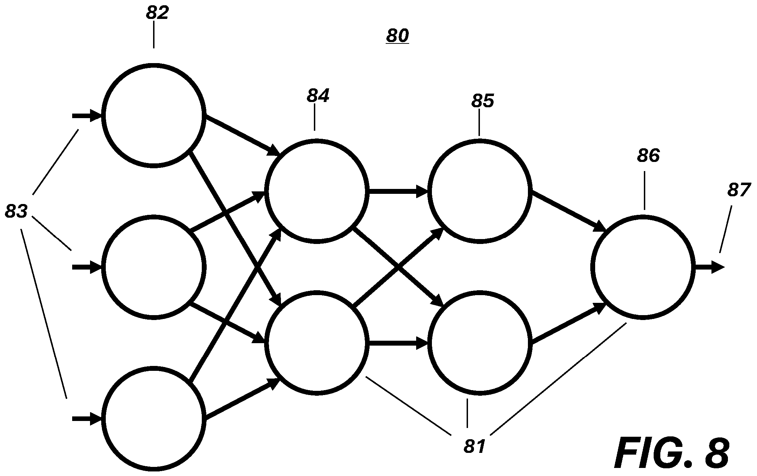

Fig. 8 depicts a machine learning (ML) model 80. ML model 80 is a computer-implemented process (or algorithm, method, function, subroutine, formula, rule etc.) which, given input data 83 identifying a mitigation plan generated for a patient (and optionally additional data for that patient including but not limited to: contextual data, severity score data, historical severity score data, and the like), produces, as an output, data 87 predicting the patient outcome expected as a consequence of following the mitigation plan. In various embodiments, for example, ML model 80 may be or comprise any one or more of a computer-implemented mathematical model, a computer-implemented statistical model, a deep learning model, and the like. In the illustrated embodiment, ML model 80 is a fully-connected "neural network", comprising a plurality of artificial neurons 81 arranged in layers, having "edges" or "connections" extending from each artificial neuron 81 in any one given (non-final) layer to every artificial neuron 81 in the subsequent layer. Artificial neurons 81 in the initial "input" layer 82 can be configured to output real numbers corresponding to encodings of data in the various fields identifying the input mitigation plan. Artificial neurons 81 in each subsequent layer (e.g., hidden layers 84, 85) receive (along each "edge") the outputs of the previous layer, combine them as a weighted sum, optionally add a bias to the total, and apply some suitable activation function. The output of this process then becomes an input to the following layer, and so forth, until a final output or set of outputs (outcome data 87) is produced by the final layer 86.

The activation function may be any suitable function including but not limited to: a step function (e.g. a binary step); a logistic, sigmoid or soft step function; a hyperbolic tangent (tanh) function, a rectified linear unit (ReLU), a "leaky" rectified linear unit, a Gaussian error linear unit (GELU), a softfplus function or any other suitable activation function as will be known to those of skill in the art. The activation function may be a nonlinear function. The final output 87 (from the last layer 86 of artificial neurons 81) may be used directly as the prediction of the patient outcome (i.e., a degree of success or failure). Alternatively, predictions may be derived from the final output by using one or more additional processing steps (e.g. rounding, truncation, normalisation, "softmax", and so forth).

ML model 80 can be "trained" using data. Training ML model 80 can comprise the use of known "pairs" of sets of data - each pair being formed of a "data point" comprising data identifying a mitigation plan generated for a patient (and optionally some or all of the abovementioned additional data), and a "label" for that data point, the label comprising data indicating received outcome data for a patient. Data points are provided to ML model 80 which uses its internal structure of parameters (e.g. weights and biases) to derive predicted labels for each of the data points. These predictions are compared to the "real" labels of the data points to determine a measure of the predictions' accuracy/inaccuracy (also referred to as "utility", "correctness", or "error/loss"), and this measure is used as feedback for adjusting/updating the values of the parameters (e.g. weights and biases) in ML model 80 to improve the quality of future predictions.

In this way the process of training ML model 80 forms probability-weighted associations between each known "input" and "result" from the corpus of data on which it is trained. Training ML model 80 can involve adjusting its weighted associations according to a learning rule and using the error value. Successive adjustments cause the neural network to produce output for a given set of mitigation plan data which is increasingly close to its corresponding patient outcome data. After a sufficient number of these adjustments, the training may be terminated.

Various suitable algorithms may be used as a learning rule for determining how to adjust the parameters of the model (e.g. weights/biases) based upon the determined total error, loss or accuracy for the prediction(s) when compared with the "true" data labels. For instance, training may comprise the use of backpropagation, stochastic gradient descent, genetic algorithms, simulated annealing, algebraic optimisation, and/or any suitable numeric or computational method for convex or non-convex optimisation known to those skilled in the art.

As the skilled person is aware, the illustrated ML model 80 is merely one of a variety of suitable implementations for an ML model, and other ML architectures and supervised, unsupervised, and semi-supervised learning paradigms will be known to the skilled artisan, including but not limited to decision trees; ensemble methods, such as random forests; k-nearest neighbour models; linear and logistic regression; Naive Bayes models; support vector machines; and other varieties of artificial neural network (recurrent neural networks, convolutional neural networks, non-fully-connected neural networks, and so forth.

Outcome data may be measured in terms e.g., of: an extent of reduction in the severity score(s) for one or more risk factors; a rate of reduction in the severity score(s) for one or more risk factors; an amount of time for which the severity score(s) for one or more risk factors are maintained constant without increasing; or any other suitable metric that can be used to quantify how well the patient has responded to the intervention(s) and the extent to which their dementia or dementia risk has improved or worsened.

Once the machine learning model has been trained, it can be used to evaluate a potential mitigation plan (a "proposed" or "candidate" plan) by providing the plan as its input (optionally alongside any other relevant contextual patient data) and taking the resultant output representing a predicted patient outcome associated with the patient following that mitigation plan. If desired, this can then be used as a means of selecting or recommending a mitigation plan for a patient - a plurality of such plans can be received and/or generated, and the trained ML model can be applied to each to obtain its predicted patient outcome. Then, whichever mitigation plan is predicted to result in the optimal patient outcome can be selected as the optimal mitigation plan (or intervention thereof, respectively).

The following exemplary scenario may help to illustrate how machine learning may be used to improve recommendations and/or provide unique advice for a user, based on data gathered from other users. The scenario may be implemented using e.g., a combination of a mobile device app and a server communicating therewith, as describe elsewhere herein.

First, data is aggregated for a plurality of patients. The aggregated data includes, inter alia, specific (optionally anonymised) personal, medical and/or situational details of each patient, such as historical severity score data, lifestyle data, previous interventions that have been recommended and their subsequent effect on severity scores, and the like. The server detects, from the aggregated data, a trend whereby patients meeting a particular profile who have been recommended a first intervention (e.g., "address your social isolation by scheduling time each day to talk to your mother") that has been unsuccessful (e.g., no change in severity score even after allowing enough time for the intervention to be adopted) nevertheless have a large degree of success (subsequent reduction in severity score) when they are recommended a second, different intervention (e.g., an event-based intervention to curb social isolation).

Subsequently, a further patient meeting the same profile is recommended the first intervention but sees no progress in reducing their social isolation severity score (e.g., due to personal dislike of their mother). The app (i.e., the client mobile device and/or server processing device) detects that the patient is likely to benefit from the event-based intervention, using the trend found in the aggregated data. The app then generates personalised suggestions for addressing the patient's elevated level of social isolation based on the locations visited by said patient (using GPS/location data or manual user input), sources events online within these locations, and provides the app user (e.g., patient) with dates that the patient can attend these events, as well as further information about what happens during these events. The app informs the patient, or their carer/clinician depending on severity of the patient's dementia, the need for an event-based intervention and creates specific recommendations of locations, events and activities for the patient to attend.

In general, when generating a mitigation plan, there may optionally be performed a further processing step in which activities listed as part of (or for use in deriving) a mitigation plan are eliminated from the list if (and/or to the extent that) those activities are likely to inflame or exacerbate other dementia risk factors of the patient (for example, pub gatherings, which may reduce social isolation at the expense of increased alcohol use).

### Miscellaneous

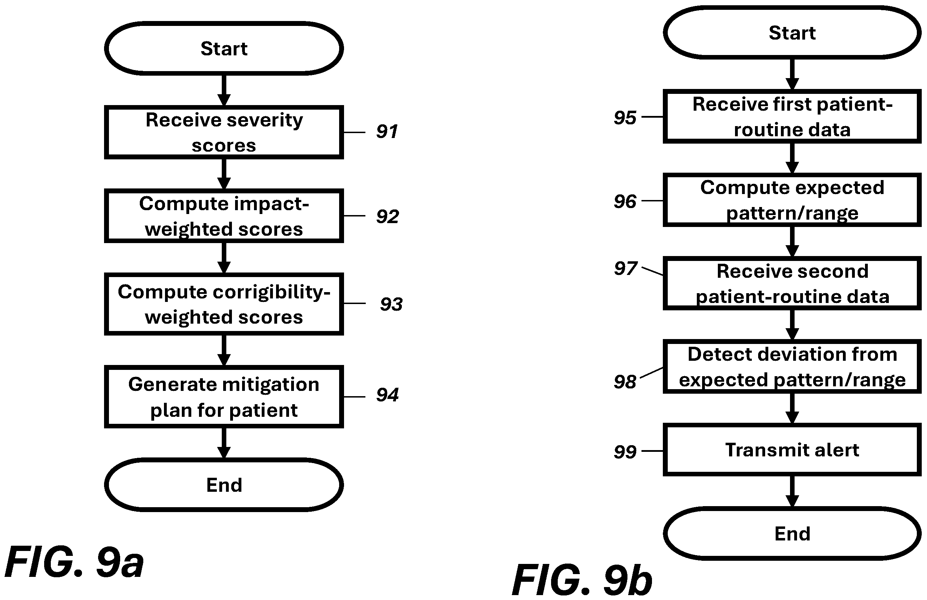

Referring now to Fig. 9a, a set of steps is provided in accordance with one aspect of the present invention.

In a step 91, a severity score is received for a patient for each of a plurality of remediable dementia risk factors.

In another step 92, an impact-weighted score is computed for the patient for each remediable dementia risk factor, based on the received severity scores and a plurality of respective predetermined impact weights.

In another step 93, a corrigibility-weighted score is computed for the patient for each remediable dementia risk factor, based on the impact-weighted scores and a plurality of respective corrigibility weights.

In another step 94, a mitigation plan is generated for the patient. Generating the mitigation plan comprises ordering the plurality of remediable dementia risk factors by their respective corrigibility-weighted scores.

Referring now to Fig. 9b, a set of steps is provided in accordance with another aspect of the present invention.

In a step 95, first patient-routine data is received during a validation period. The first patient-routine data comprises location data and/or GPS data determined by a wearable device or mobile device of the patient.

In another step 96, an expected pattern or range is computed for the patient for each of a plurality of patient-routine metrics, based on the first patient-routine data.

In another step 97, second patient-routine data is received after expiry of the validation period. The second patient-routine data comprises location data and/or GPS data determined by a wearable device or mobile device of the patient.

In another step 98, a deviation from the expected pattern or range in one or more of the patient-routine metrics is detected based on the second patient-routine data.

In another step 99, an alert is transmitted to a clinician based on the detected deviation.

The term "comprising" encompasses "including" as well as "consisting" e.g. a composition "comprising" X may consist exclusively of X or may include something additional e.g. X + Y.

Unless otherwise indicated each embodiment as described herein may be combined with another embodiment as described herein.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, hard-drives, thumb drives, memory cards, etc. and do not include propagated signals. The software can be suitable for execution on a parallel processor or a serial processor such that the method steps may be carried out in any suitable order, or simultaneously. This acknowledges that firmware and software can be valuable, separately tradable commodities. It is intended to encompass software, which runs on or controls "dumb" or standard hardware, to carry out the desired functions. It is also intended to encompass software which "describes" or defines the configuration of hardware, such as HDL (hardware description language) software, as is used for designing silicon chips, or for configuring universal programmable chips, to carry out desired functions.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilizing conventional techniques known to those skilled in the art that all, or a portion of the software instructions may be carried out by a dedicated circuit, such as a DSP (Digital Signal Processor), programmable logic array, or the like.

It will be understood that the benefits and advantages described above may relate to one embodiment or may relate to several embodiments. The embodiments are not limited to those that solve any or all of the stated problems or those that have any or all of the stated benefits and advantages.

The steps of the methods described herein may be carried out in any suitable order, or simultaneously where appropriate. Additionally, individual steps may be deleted from any of the methods without departing from the spirit and scope of the subject matter described herein. Aspects of any of the examples described above may be combined with aspects of any of the other examples described to form further examples without losing the effect sought. Any of the steps or processes described above may be implemented in hardware or software unless otherwise specified.

It will be understood that the above descriptions of preferred embodiments are given by way of example only and that various modifications are possible within the scope of the appended claims and may be made by those skilled in the art. Although various embodiments have been described above with a certain degree of particularity, or with reference to one or more individual embodiments, those skilled in the art could make numerous alterations to the disclosed embodiments without departing from the scope of this disclosure.

All trademarks, and all product, service, company and brand names are property of their respective owners and are used herein for illustrative purposes only. Reasonable efforts have been made to capitalise all such trademarks herein. Use of these names, marks, and brands does not imply endorsement.

The following numbered list of Examples forms part of the present disclosure:
1. A computer program comprising instructions which, when the program is executed by a computer, cause the computer to carry out steps comprising:
   receiving, for a patient, a severity score for each of a plurality of remediable dementia risk factors;
   computing, for the patient, an impact-weighted score for each remediable dementia risk factor based on the received severity scores and a plurality of respective predetermined impact weights;
   computing, for the patient, a corrigibility-weighted score for each remediable dementia risk factor based on the impact-weighted scores and a plurality of respective corrigibility weights; and
   generating a mitigation plan for the patient, wherein generating the mitigation plan comprises ordering the plurality of remediable dementia risk factors by their respective corrigibility-weighted scores.
2. A device comprising a processor and a memory, the memory comprising instructions which, when executed by the processor, cause the processor to carry out steps comprising:
   receiving, for a patient, a severity score for each of a plurality of remediable dementia risk factors;
   computing, for the patient, an impact-weighted score for each remediable dementia risk factor based on the received severity scores and a plurality of predetermined impact weights;
   computing, for the patient, a corrigibility-weighted score for each remediable dementia risk factor based on the impact-weighted scores and a plurality of corrigibility weights; and
   generating a mitigation plan for the patient, wherein generating the mitigation plan comprises ordering the plurality of remediable dementia risk factors by their respective corrigibility-weighted scores.
3. A computer-implemented method comprising the following steps:
   receiving, for a patient, a severity score for each of a plurality of remediable dementia risk factors;
   computing, for the patient, an impact-weighted score for each remediable dementia risk factor based on the received severity scores and a plurality of predetermined impact weights;
   computing, for the patient, a corrigibility-weighted score for each remediable dementia risk factor based on the impact-weighted scores and a plurality of corrigibility weights; and
   generating a mitigation plan for the patient, wherein generating the mitigation plan comprises ordering the plurality of remediable dementia risk factors by their respective corrigibility-weighted scores.
4. The program of Example 1, device of Example 2, or method of Example 3 wherein the severity scores represent, for each remediable dementia risk factor, an extent to which the patient exhibits the risk factor.
5. The program, device, or method of any preceding Example, wherein generating the mitigation plan comprises one or more of:
   generating, for one or more of the remediable dementia risk factors, a recommendation of a lifestyle change for the patient to mitigate the respective one or more risk factors based on the ordering and/or the respective corrigibility-weighted scores;
   generating an action plan based on the ordering and based on one or more identified actions for addressing at least one risk factor of the plurality of remediable dementia risk factors; or
   determining one or more prioritised risk factors among the remediable dementia risk factors based on the ordering and/or the respective corrigibility-weighted scores.
6. The program, device, or method of any preceding Example, wherein receiving the severity scores comprises:
   receiving, for the patient, a severity score for each of a plurality of dementia risk factors, said plurality including each of the remediable dementia risk factors and one or more irremediable dementia risk factors; and
   filtering the plurality of received severity scores to retain only the severity scores for the remediable dementia risk factors.
7. The program, device, or method of any preceding Example, wherein the remediable dementia risk factors include one or more of: hearing loss, LDL cholesterol level, social isolation, depression, anxiety, physical inactivity, diabetes, smoking, hypertension, vision loss, obesity, and alcohol use.
8. The program, device, or method of any preceding Example, wherein the predetermined impact weights are provided in a ratio of: about 7 for hearing loss; about 7 for LDL cholesterol level; about 5 for social isolation; about 3 for depression; about 3 for anxiety; about 2 for physical inactivity; about 2 for diabetes; about 2 for smoking; about 2 for hypertension; about 2 for vision loss; about 1 for obesity; and about 1 for alcohol use.
9. The program, device, or method of any preceding Example, wherein the impact weights represent, for each remediable dementia risk factor, the statistical contribution of said risk factor to a likelihood of the patient developing dementia, optionally wherein the impact weights are derived from statistical population data.
10. The program, device, or method of any preceding Example, wherein the corrigibility weights represent the extent to which the respective factors are capable of correction for the patient.
11. The program, device, or method of any preceding Example, wherein the corrigibility weights comprise, for each remediable dementia risk factor, a factor for a level of psychological effort and/or a factor for a level of logistical feasibility associated with adoption of an intervention by the patient to mitigate said risk factor, optionally wherein the factor for the level of psychological effort and/or the factor for the level of logistical feasibility are input by the patient or a care provider thereof, or are predetermined.
12. The program, device, or method of any preceding Example, wherein the steps further comprise outputting to a display one or more of:
   the generated mitigation plan;
   the received severity scores for one or more of the remediable dementia risk factors;
   the calculated impact-weighted scores for one or more of the remediable dementia risk factors;
   the calculated corrigibility-weighted scores for one or more of the remediable dementia risk factors;
   a total remediable risk score based on adding the severity scores for each of the plurality of remediable dementia risk factors;
   a total impact-weighted risk score based on adding the impact-weighted scores for each of the plurality of remediable dementia risk factors;
   the received severity scores for one or more of the irremediable dementia risk factors; and
   a total risk score based on adding the severity scores for each of the remediable dementia risk factors and each of the irremediable dementia risk factors.
13. The program, device, or method of Example 12, wherein said outputting comprises generating and rendering one or both of:
   a chart representing the severity scores for each of the remediable dementia risk factors; and
   a graph representing a change over time for the severity score of each of one or more of the remediable dementia risk factors.
14. The program, device, or method of any preceding Example, wherein the steps further comprise:
   receiving, for the patient, an updated severity score for one or more selected risk factors of the plurality of remediable dementia risk factors;
   computing an impact-weighted score for each selected risk factor based on the one or more updated severity scores and the plurality of impact weights; and
   computing a total impact-weighted risk score based on adding the impact-weighted scores for each of the selected risk factors to the impact-weighted scores for each of the remaining remediable dementia risk factors;
   optionally wherein the steps further comprise providing an output to a display based on:
   said computed total impact-weighted risk score; and
   a second total impact-weighted risk score based on adding the impact-weighted scores for each of the plurality of remediable dementia risk factors.
15. The program, device, or method of any preceding Example, wherein the steps further comprise:
   generating a hypothetical reduced severity score for one or more selected risk factors of the plurality of remediable dementia risk factors;
   computing an impact-weighted score for each selected risk factor based on the one or more hypothetical reduced severity scores and the plurality of impact weights; and
   computing a hypothetical total impact-weighted risk score based on adding the impact-weighted scores for each of the selected risk factors to the impact-weighted scores for each of the remaining remediable dementia risk factors;
   optionally wherein the steps further comprise providing an output to a display based on:
   said computed hypothetical total impact-weighted risk score; and
   a second total impact-weighted risk score based on adding the impact-weighted scores for each of the plurality of remediable dementia risk factors.
16. The program, device, or method of any preceding Example, wherein the received severity scores comprise:
   severity scores received from electronic medical records; and/or
   severity scores received based on input to a graphical user interface by the patient or a caregiver thereof.
17. The program, device, or method of any preceding Example, wherein one or more of the severity scores are generated based on:
   biometric data sensed by a wearable device; and/or
   location data and/or GPS data determined by a wearable device or mobile device.
18. The program, device, or method of Example 17, wherein receiving the severity scores comprises generating a severity score for a social isolation risk factor, wherein generating said severity score comprises:
   receiving patient-routine data comprising location data and/or GPS data determined by a wearable device or mobile device of the patient;
   deriving one or more patient-routine metrics from the received patient-routine data; and
   optionally generating and outputting one or more patient prompts based on the derived patient-routine metrics and receiving one or more patient responses to said patient prompts.
19. The program, device, or method of Example 18, wherein generating the mitigation plan further comprises:
   for each candidate activity of a plurality of candidate activities, comparing location data for said candidate activity against the derived patient-routine metrics to determine a fit score; and
   incorporating one or more of the candidate activities into the generated mitigation plan based on their determined fit score and the generated severity score for the social isolation risk factor; optionally wherein the plurality of candidate activities are generated based on the patient-routine data and/or the derived patient-routine metrics.
20. The program, device, or method of any preceding Example, wherein generating the mitigation plan comprises generating an intervention for at least one risk factor, based on one or both of:
   the severity score for said risk factor; and
   an amount of time in which the severity score for said risk factor has remained unchanged.
21. The program, device, or method of any preceding Example, wherein the steps further comprise:
   detecting attempts by the patient to access or view a patient data item;
   computing a frequency or volume of the detected attempts;
   comparing said frequency or volume to a predetermined threshold; and
   upon determination that the frequency or volume exceeds the predetermined threshold, triggering an anti-obsession action.
22. The program, device, or method of Example 21, wherein the patient data item comprises one or more of:
   one of the received severity scores;
   one of the computed impact-weighted scores;
   one of the computed corrigibility-weighted scores;
   the total remediable risk score;
   the total impact-weighted risk score;
   the total risk score;
   the hypothetical total impact-weighted risk score;
   the chart; or
   the graph.
23. The program, device, or method of Example 21 or 22, wherein the anti-obsession action comprises one or more of:
   transmitting an alert to a clinician,
   outputting an alert to the patient,
   blocking the attempt, or
   locking out the patient.
24. The method of any one of Examples 3 to 23, wherein the severity scores are received by a server device, the impact-weighted scores are computed by the server device, the corrigibility-weighted scores are computed by the server device, and the mitigation plan is generated by the server device; and wherein the steps further comprise transmitting the mitigation plan from the server device to a client device.
25. The program, device, or method of any preceding Example, wherein the steps comprise repeating said receiving the severity scores, computing the impact-weighted scores, computing the corrigibility-weighted scores, and generating the mitigation plan for each patient of a plurality of patients; and wherein the steps further comprise:
   receiving outcome data for each patient of the plurality of patients;
   generating a training dataset by matching mitigation plans or interventions thereof to the outcome data; and
   training a supervised machine learning model to map mitigation plans or interventions thereof to predicted outcomes using the training dataset.
26. The program, device, or method of Example 25, wherein the steps further comprise using the trained supervised machine learning model to predict an outcome of each of one or more proposed mitigation plans or interventions thereof, and optionally to output a recommended mitigation plan or intervention thereof based on the predicted outcomes.
27. A computer program comprising instructions which, when the program is executed by a computer, cause the computer to carry out steps comprising:
   receiving first patient-routine data during a validation period, the first patient-routine data comprising location data and/or GPS data determined by a wearable device or mobile device of the patient;
   computing an expected pattern or range for the patient for each of a plurality of patient-routine metrics, based on the first patient-routine data;
   receiving second patient-routine data after expiry of the validation period, the second patient-routine data comprising location data and/or GPS data determined by a wearable device or mobile device of the patient;
   detecting a deviation from the expected pattern or range in one or more of the patient-routine metrics based on the second patient-routine data; and
   transmitting an alert to the patient and/or a clinician based on the detected deviation.
28. A device comprising a processor and a memory, the memory comprising instructions which, when executed by the processor, cause the processor to:
   receive first patient-routine data during a validation period, the first patient-routine data comprising location data and/or GPS data determined by a wearable device or mobile device of the patient;
   compute an expected pattern or range for the patient for each of a plurality of patient-routine metrics, based on the first patient-routine data;
   receive second patient-routine data after expiry of the validation period, the second patient-routine data comprising location data and/or GPS data determined by a wearable device or mobile device of the patient;
   detect a deviation from the expected pattern or range in one or more of the patient-routine metrics based on the second patient-routine data; and
   transmit an alert to a clinician based on the detected deviation.
29. A computer-implemented method comprising:
   receiving first patient-routine data during a validation period, the first patient-routine data comprising location data and/or GPS data determined by a wearable device or mobile device of the patient;
   computing an expected pattern or range for the patient for each of a plurality of patient-routine metrics, based on the first patient-routine data;
   receiving second patient-routine data after expiry of the validation period, the second patient-routine data comprising location data and/or GPS data determined by a wearable device or mobile device of the patient;
   detecting a deviation from the expected pattern or range in one or more of the patient-routine metrics based on the second patient-routine data; and
   transmitting an alert to a clinician based on the detected deviation.
30. The program of Example 27, device of Example 28, or method of Example 29, wherein the patient-routine metrics comprise one or more of:
   a route taken between two locations;
   an amount of time taken to travel between two locations;
   a frequency with which a location is visited;
   an amount of time spent at a location;
   a day or plurality of days on which a location is visited; and
   a time or range of times at/in which a location is visited.
31. A non-transitory computer-readable storage medium containing instructions which, when read by a computer, cause the computer to perform the method of Example 3, Example 29, or Example 30.

## Claims

1. A computer-implemented method comprising the following steps:
receiving, for a patient, a severity score for each of a plurality of remediable dementia risk factors;
computing, for the patient, an impact-weighted score for each remediable dementia risk factor based on the received severity scores and a plurality of predetermined impact weights;
computing, for the patient, a corrigibility-weighted score for each remediable dementia risk factor based on the impact-weighted scores and a plurality of corrigibility weights; and
generating a mitigation plan for the patient, wherein generating the mitigation plan comprises ordering the plurality of remediable dementia risk factors by their respective corrigibility-weighted scores.

2. The method of claim 1, wherein generating the mitigation plan comprises one or more of:
generating, for one or more of the remediable dementia risk factors, a recommendation of a lifestyle change for the patient to mitigate the respective one or more risk factors based on the ordering and/or the respective corrigibility-weighted scores;
generating an action plan based on the ordering and based on one or more identified actions for addressing at least one risk factor of the plurality of remediable dementia risk factors;
determining one or more prioritised risk factors among the remediable dementia risk factors based on the ordering and/or the respective corrigibility-weighted scores; or
generating an intervention for at least one risk factor, based on one or both of:
the severity score for said risk factor; and
an amount of time in which the severity score for said risk factor has remained unchanged.

3. The method of claim 1 or claim 2, wherein the steps further comprise outputting to a display one or more of:
the generated mitigation plan;
the received severity scores for one or more of the remediable dementia risk factors;
the calculated impact-weighted scores for one or more of the remediable dementia risk factors;
the calculated corrigibility-weighted scores for one or more of the remediable dementia risk factors;
a total remediable risk score based on adding the severity scores for each of the plurality of remediable dementia risk factors;
a total impact-weighted risk score based on adding the impact-weighted scores for each of the plurality of remediable dementia risk factors;
the received severity scores for one or more of the irremediable dementia risk factors; and
a total risk score based on adding the severity scores for each of the remediable dementia risk factors and each of the irremediable dementia risk factors;
optionally wherein said outputting comprises generating and rendering one or both of:
a chart representing the severity scores for each of the remediable dementia risk factors; and
a graph representing a change over time for the severity score of each of one or more of the remediable dementia risk factors.

4. The method of any preceding claim, wherein the steps further comprise:
receiving, for the patient, an updated severity score for one or more selected risk factors of the plurality of remediable dementia risk factors;
computing an impact-weighted score for each selected risk factor based on the one or more updated severity scores and the plurality of impact weights; and
computing a total impact-weighted risk score based on adding the impact-weighted scores for each of the selected risk factors to the impact-weighted scores for each of the remaining remediable dementia risk factors;
optionally wherein the steps further comprise providing an output to a display based on:
said computed total impact-weighted risk score; and
a second total impact-weighted risk score based on adding the impact-weighted scores for each of the plurality of remediable dementia risk factors.

5. The method of any preceding claim, wherein the steps further comprise:
generating a hypothetical reduced severity score for one or more selected risk factors of the plurality of remediable dementia risk factors;
computing an impact-weighted score for each selected risk factor based on the one or more hypothetical reduced severity scores and the plurality of impact weights; and
computing a hypothetical total impact-weighted risk score based on adding the impact-weighted scores for each of the selected risk factors to the impact-weighted scores for each of the remaining remediable dementia risk factors;
optionally wherein the steps further comprise providing an output to a display based on:
said computed hypothetical total impact-weighted risk score; and
a second total impact-weighted risk score based on adding the impact-weighted scores for each of the plurality of remediable dementia risk factors.

6. The method of any preceding claim, wherein one or more of the severity scores are generated based on:
biometric data sensed by a wearable device; and/or
location data and/or GPS data determined by a wearable device or mobile device.

7. The method of claim 6, wherein receiving the severity scores comprises generating a severity score for a social isolation risk factor, wherein generating said severity score comprises:
receiving patient-routine data comprising location data and/or GPS data determined by a wearable device or mobile device of the patient;
deriving one or more patient-routine metrics from the received patient-routine data; and
optionally generating and outputting one or more patient prompts based on the derived patient-routine metrics and receiving one or more patient responses to said patient prompts.

8. The method of claim 7, wherein generating the mitigation plan further comprises:
for each candidate activity of a plurality of candidate activities, comparing location data for said candidate activity against the derived patient-routine metrics to determine a fit score; and
incorporating one or more of the candidate activities into the generated mitigation plan based on their determined fit score and the generated severity score for the social isolation risk factor; optionally wherein the plurality of candidate activities are generated based on the patient-routine data and/or the derived patient-routine metrics.

9. The method of any preceding claim, wherein the steps further comprise:
detecting attempts by the patient to access or view a patient data item;
computing a frequency or volume of the detected attempts;
comparing said frequency or volume to a predetermined threshold; and
upon determination that the frequency or volume exceeds the predetermined threshold, triggering an anti-obsession action.

10. The method of any preceding claim, wherein the steps comprise repeating said receiving the severity scores, computing the impact-weighted scores, computing the corrigibility-weighted scores, and generating the mitigation plan for each patient of a plurality of patients; and wherein the steps further comprise:
receiving outcome data for each patient of the plurality of patients;
generating a training dataset by matching mitigation plans or interventions thereof to the outcome data; and
training a supervised machine learning model to map mitigation plans or interventions thereof to predicted outcomes using the training dataset;
optionally wherein the steps further comprise:
using the trained supervised machine learning model to predict an outcome of each of one or more proposed mitigation plans or interventions thereof, and optionally to output a recommended mitigation plan or intervention thereof based on the predicted outcomes.

11. The method of any preceding claim, wherein the severity scores are received by a server device, the impact-weighted scores are computed by the server device, the corrigibility-weighted scores are computed by the server device, and the mitigation plan is generated by the server device; and wherein the steps further comprise transmitting the mitigation plan from the server device to a client device.

12. A computer-implemented method comprising:
receiving first patient-routine data during a validation period, the first patient-routine data comprising location data and/or GPS data determined by a wearable device or mobile device of the patient;
computing an expected pattern or range for the patient for each of a plurality of patient-routine metrics, based on the first patient-routine data;
receiving second patient-routine data after expiry of the validation period, the second patient-routine data comprising location data and/or GPS data determined by a wearable device or mobile device of the patient;
detecting a deviation from the expected pattern or range in one or more of the patient-routine metrics based on the second patient-routine data; and
transmitting an alert to a clinician based on the detected deviation.

13. A non-transitory computer-readable storage medium containing instructions which, when read by a computer, cause the computer to perform the method of any one of claims 1 to 10 or claim 12.

14. A computer program comprising instructions which, when the program is executed by a computer, cause the computer to carry out the method of any one of claims 1 to 10 or claim 12.

15. A device comprising a processor and a memory, the memory comprising instructions which, when executed by the processor, cause the processor to carry out the method of any one of claims 1 to 10 or claim 12.
